(19) Europäisches Patentamt
European Patent Office
Office européen des brevets

(11) EP 3 980 001 B1

(12) EUROPEAN PATENT SPECIFICATION

(45) Date of publication and mention of the grant of the patent:
**27.08.2025 Bulletin 2025/35**

(21) Application number: **20822078.0**

(22) Date of filing: **10.06.2020**

(51) International Patent Classification (IPC):
***A61K 9/20** (2006.01)* ***A61K 31/194** (2006.01)*
***A61K 33/06** (2006.01)* ***A61K 9/22** (2006.01)*
***A61K 9/00** (2006.01)* ***A61K 9/48** (2006.01)*
***A61K 47/38** (2006.01)*

(52) Cooperative Patent Classification (CPC):
**A61K 33/06; A61K 9/0053; A61K 9/2054; A61K 9/4866; A61K 31/194; A61K 47/38**

(86) International application number:
**PCT/US2020/036987**

(87) International publication number:
**WO 2020/252014 (17.12.2020 Gazette 2020/51)**

# (54) SUSTAINED-RELEASE COMPOSITIONS OF ALPHA-KETOGLUTARATE

ZUSAMMENSETZUNGEN VON ALPHA-KETOGLUTARAT VERZÖGERTER FREISETZUNG

COMPOSITIONS À LIBÉRATION PROLONGÉE D'ALPHA-CÉTOGLUTARATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2019 US 201962859595 P**
**31.10.2019 US 201962929024 P**

(43) Date of publication of application:
**13.04.2022 Bulletin 2022/15**

(73) Proprietor: **Ponce de Leon Health Designated Activity Company**
**Drogheda, Louth (IE)**

(72) Inventors:
- **PEREIRA, David Eugene**
**Apex, North Carolina 27502 (US)**
- **KONAR, Sumana**
**Dehradun 248110 (IN)**

(74) Representative: **Thurston, Joanna**
**Withers & Rogers LLP**
**2 London Bridge**
**London SE1 9RA (GB)**

(56) References cited:
**WO-A1-2013/160792** **WO-A2-2018/200736**
**MX-A- 2017 004 010** **US-A1- 2004 053 818**
**US-A1- 2005 106 246** **US-A1- 2009 005 437**

- **HOGAN J: "Hydroxypropylmethylcellulose sustained release technology", JOURNAL DRUG DEVELOPMENT AND INDUSTRIAL PHARMACY, NEW YORK, NY, US, vol. 15, no. 6, 1 January 1989 (1989-01-01), pages 975 - 999, XP009119167, ISSN: 0363-9045, DOI: 10.3109/03639048909043660**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 980 001 B1

## Description

BACKGROUND

Alpha-ketoglutaric acid is an important biological molecule, being a key intermediate in the Krebs cycle, a nitrogen transporter, and a co-substance in molecular oxidation. Alpha-ketoglutaric acid anion plays a key role in metabolism, mainly in aerobic organisms. A calcium salt of alpha-ketoglutarate (Ca-AKG) is an important source of alpha-ketoglutaric acid anion. Currently available Ca-AKG is formulated for immediate release. Such known delivery methods release Ca-AKG within a small period of time which leads to a spike in blood concentration, faster excretion, and increased frequency of administration of the Ca-AKG. Accordingly, there is a need for discovery of alternative methods for the delivery of Ca-AKG. Patent document WO 2018/200736 discloses a method of extending lifespan in a subject in need thereof comprising administering to the subject a therapeutically effective amount of berberine, a vitamin A compound, and a-ketoglutarate (AKG).

SUMMARY OF THE INVENTION

It has been unexpectedly discovered that when alpha-ketoglutarates are released in the stomach or early in the upper gastrointestinal (GI) tract, the overall bioavailability of the alpha-ketoglutarates are variable and/or low, leading to less predictable dosing guidance, and less predictable clinical outcomes. A controlled release, sustained release, and/or delayed release formulation of Ca-AKG has been discovered. Described herein are formulations of alpha-ketoglutarates having modified release characteristics, including controlled release, sustained release, and/or delayed release characteristics. In one illustrative embodiment of the invention, compositions comprising

(a) a therapeutically effective amount of calcium alpha-ketoglutarate, and
(b) a controlled-release matrix, wherein the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000, are described herein. Illustratively, the composition is characterized by, capable of, or configured to provide a release rate of the calcium alpha-ketoglutarate of no more than 90% of a release rate of the calcium alpha-ketoglutarate of one or more calcium alpha-ketoglutarate compositions that are formulated without any controlled-release matrix, or where the active ingredients are otherwise released in an uncontrolled manner, over or during a predetermined period of time. Illustrative examples of calcium alpha-ketoglutarate compositions, for comparison, that are formulated without any controlled-release matrix include, but are not limited to, Simplesa™ and Klaire Labs™.

In another embodiment, also disclosed herein are compositions comprising calcium alpha-ketoglutarate, and one or more agents selected from the group consisting of surfactants, preservatives, flavoring agents, vitamins, antioxidants, sweetening agents, and combinations thereof.

In another embodiment, also disclosed herein are compositions comprising:

525 mg of calcium alpha-ketoglutarate monohydrate;
one or more release-modifying agents;

and optionally further comprising one or more of isomalt, vegetable wax (carnauba and/or rice bran), stearic acid, magnesium stearate, and silica.

In another embodiment, also disclosed herein are compositions comprising:

500-550 mg of calcium alpha-ketoglutarate monohydrate;
450 mcg of retinyl palmitate;
one or more release-modifying agents;

and optionally further comprising one or more of isomalt, vegetable wax (carnauba and/or rice bran), stearic acid, magnesium stearate, and silica.

In another embodiment, also disclosed herein are compositions comprising:

500-550 mg of calcium alpha-ketoglutarate monohydrate;
12.5 mcg (500 IU) of cholecalciferol;

one or more release-modifying agents;

and optionally further comprising one or more of isomalt, vegetable wax (carnauba and/or rice bran), stearic acid, magnesium stearate, and silica.

It is to be understood that in the formulations described herein, such release-modifying agents may be used to control, delay, or sustain the release of Ca-AKG, or to control, delay, or sustain the release of a combination of Ca-AKG and one or more of the additional components of the composition, such as a combination of Ca-AKG and one or more vitamins.

It has been surprisingly discovered that compared to alternative formulations that are formulated without any controlled-release matrix, or where the active ingredients are otherwise released in an uncontrolled manner, the formulations described herein may include lower doses of active ingredients, including lower doses of AKG, and/or salts thereof. Without being bound by theory, it is believed herein that formulations described herein provide higher overall bioavailability, and therefore, lower doses can be administered to achieve the similar outcomes.

## DETAILED DESCRIPTION OF THE DISCLOSURE

Illustrative embodiments of the invention are described in the following clauses:

A composition, including a unit dose or dosage form, the composition comprising (a) a therapeutically effective amount of calcium alpha-ketoglutarate, and (b) a controlled-release matrix.

The composition, including the unit dose or dosage form, of the preceding clause wherein the controlled-release matrix comprises hydroxypropylmethylcellulose (HPMC).

The composition, including the unit dose or dosage form, of any one of the preceding clauses wherein the hydroxypropylmethylcellulose (HPMC) is selected from the group consisting of HPMC with number average molecular weight of about 22,000-30,000, about 68,000- 95,000, about 115,000-150,000, and about 220,000-300,000, and combinations thereof. The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

The composition, including the unit dose or dosage form, of any one of the preceding clauses wherein the controlled-release matrix comprises HPMC with number average molecular weight of about 68,000-95,000.

The composition, including the unit dose or dosage form, of any one of the preceding clauses wherein the controlled-release matrix comprises HPMC with number average molecular weight of about 115,000-150,000.

The composition, including the unit dose or dosage form, of any one of the preceding clauses wherein the controlled-release matrix comprises HPMC with number average molecular weight of between about 68,000-95,000 and about 115,000-150,000.

The composition, including the unit dose or dosage form, of any one of the preceding clauses wherein the amount of calcium alpha-ketoglutarate is from about 30% to about 65% by total weight of the composition; from about 40% to about 65% by total weight of the composition; from about 40% to about 60% by total weight of the composition; or from about 45% to about 55% by total weight of the composition.

The composition, including the unit dose or dosage form, of any one of the preceding clauses wherein the amount of calcium alpha-ketoglutarate is about 30%, about 35%, about 40%, about 45%, about 50%, about 55%, about 60%, or about 65%, by total weight of the composition.

The composition, including the unit dose or dosage form, of any one of the preceding clauses comprising about 250 to about 1000 mg of calcium alpha-ketoglutarate; about 350 to about 1000 mg of calcium alpha-ketoglutarate; about 400 to about 1000 mg of calcium alpha-ketoglutarate; about 400 to about 900 mg of calcium alpha-ketoglutarate; about 400 to about 800 mg of calcium alpha-ketoglutarate; about 400 to about 700 mg of calcium alpha-ketoglutarate; or about 400 to about 600 mg of calcium alpha-ketoglutarate.

The composition, including the unit dose or dosage form, of any one of the preceding clauses comprising about 400 mg of calcium alpha-ketoglutarate; about 425 mg of calcium alpha-ketoglutarate; about 450 mg of calcium alpha-ketoglutarate; about 500 mg of calcium alpha-ketoglutarate; about 525 mg of calcium alpha-ketoglutarate; about 550 mg of calcium alpha-ketoglutarate; about 575 mg of calcium alpha-ketoglutarate; or about 600 mg of calcium alpha-ketoglutarate.

The composition, including the unit dose or dosage form, of any one of the preceding clauses comprising about 5% dietary value of calcium; about 6% dietary value of calcium; about 7% dietary value of calcium; about 8% dietary value of calcium; about 9% dietary value of calcium; about 10% dietary value of calcium; about 11% dietary value of calcium; about 12% dietary value of calcium; about 13% dietary value of calcium; about 14% dietary value of calcium; about 15% dietary value of calcium; about 16% dietary value of calcium; about 17% dietary value of calcium; about 18% dietary value of calcium; about 19% dietary value of calcium; about 20% dietary value of calcium.

The composition, including the unit dose or dosage form, of any one of the preceding clauses further comprising one or more excipients; selected from the group consisting of one or more diluents, fillers, disintegrants, lubricants, binders, and suspending agents, and combinations thereof.

The composition, including the unit dose or dosage form, of any one of the preceding clauses further comprising microcrystalline cellulose; wherein the amount of the microcrystalline cellulose is from about 14% to about 27% by total weight of the composition; about 17% about to 23% by total weight of the composition; about 17% to about 22% by total weight of the composition; from about 17% to about 20% by total weight of the composition.

The composition, including the unit dose or dosage form, of any one of the preceding clauses further comprising lactose monohydrate; the amount of the lactose monohydrate is from about 6% to about 19% by total weight of composition; about 8% to about 17% by total weight of composition; about 10% to about 15% by total weight of composition; about 11% to about 14% by total weight of composition.

The composition, including the unit dose or dosage form, of any one of the preceding clauses further comprising one or more lubricants; wherein the lubricant is magnesium stearate; wherein the amount of magnesium stearate is from about 1% to about 5% by total weight of composition; about 1% to about 3% by total weight of composition; about 2% to about 4% by total weight of composition.

The composition, including the unit dose or dosage form, of any one of the preceding clauses further comprising one or more disintegrants; wherein the disintegrant is silicon dioxide; wherein the amount of silicon dioxide is from about 1% to about 5% by total weight of composition; about 1% to about 3% by total weight of composition; about 2% to about 4% by total weight of composition.

The composition, including the unit dose or dosage form, of any one of the preceding clauses further comprising a sweetener; wherein the sweetener is isomalt.

The composition, including the unit dose or dosage form, of any one of the preceding clauses further comprising wax, wherein the wax is carnauba wax and/or rice bran wax.

The composition, including the unit dose or dosage form, of any one of the preceding clauses further comprising one or more agents selected from the group consisting of surfactants, preservatives, flavoring agents, vitamins, antioxidants, and sweetening agents.

The composition, including the unit dose or dosage form, of any one of the preceding clauses further comprising one or more vitamins or analogs or derivatives thereof, wherein the vitamin is nicotinamide riboside (NR); wherein the vitamin is nicotinamide mononucleotide (NMN).

The composition, including the unit dose or dosage form, of any one of the preceding clauses further comprising an antioxidant; wherein the antioxidant is fisetin.

The composition, including the unit dose or dosage form, of any one of the preceding clauses wherein the composition is characterized by a release rate of the calcium alpha-ketoglutarate that is about 90% or less of a release rate of calcium alpha-ketoglutarate of a calcium alpha-ketoglutarate composition that is uncontrolled or is formulated without the controlled-release matrix; about 70% or less of a release rate of calcium alpha-ketoglutarate of a calcium alpha-ketoglutarate composition that is uncontrolled or is formulated without the controlled-release matrix; 60% or less of a release rate of calcium alpha-ketoglutarate of a calcium alpha-ketoglutarate composition that is uncontrolled or is formulated without the controlled-release matrix; about 50% or less of a release rate of calcium alpha-ketoglutarate of a calcium alpha-ketoglutarate composition that is uncontrolled or is formulated without the controlled-release matrix; about 40% or less of a release rate of calcium alpha-ketoglutarate of a calcium alpha-ketoglutarate composition that is uncontrolled or is formulated without the controlled-release matrix; about 45% or less of a release rate of calcium alpha-ketoglutarate of a calcium alpha-ketoglutarate composition that is uncontrolled or is formulated without the controlled-release matrix; about 30% or less of a release rate of calcium alpha-ketoglutarate of a calcium alpha-ketoglutarate composition that is uncontrolled or is formulated without the controlled-release matrix.

The composition, including the unit dose or dosage form, of any one of the preceding clauses wherein the composition is characterized by a release rate of the calcium alpha-ketoglutarate that is about 30% or less after a time between about 0.5 and about 2 hours in a dissolution medium as measured by a paddle apparatus with a basket rotating at 75 rpm wherein the dissolution medium is 900 mL of about 0.03 M NaCl and about 0.08 M HCl at about 37°C; about 25% or less after a time between about 0.5 and about 1.5 hours in a dissolution medium as measured by a paddle apparatus with a basket rotating at 75 rpm wherein the dissolution medium is 900 mL of about 0.03 M NaCl and about 0.08 M HCl at about 37°C; about 25% or less after about 1 hour in a dissolution medium as measured by a paddle apparatus with a basket rotating at 75 rpm wherein the dissolution medium is 900 mL of about 0.03 M NaCl and about 0.08 M HCl at about 37°C; about 20% or less after about 1 hour in a dissolution medium as measured by a paddle apparatus with a basket rotating at 75 rpm wherein the dissolution medium is 900 mL of about 0.03 M NaCl and about 0.08 M HCl at about 37°C; about 60% or less after a time between about 4 and about 8 hours in a dissolution medium as measured by a paddle apparatus with a basket rotating at 75 rpm wherein the dissolution medium is 900 mL of about 0.03 M NaCl and about 0.08 M HCl at about 37°C; about 60% or less after a time of about 8 hours in a dissolution medium as measured by a paddle apparatus with a basket rotating at 75 rpm wherein the dissolution medium is 900 mL of about 0.03 M NaCl and about 0.08 M HCl at about

37°C; about 60% or less after a time between about 4 and about 6 hours in a dissolution medium as measured by a paddle apparatus with a basket rotating at 75 rpm wherein the dissolution medium is 900 mL of about 0.03 M NaCl and about 0.08 M HCl at about 37°C; or about 60% or less after a time of about 6 hours in a dissolution medium as measured by a paddle apparatus with a basket rotating at 75 rpm wherein the dissolution medium is 900 mL of about 0.03 M NaCl and about 0.08 M HCl at about 37°C.

The composition, including the unit dose or dosage form, of any one of the preceding clauses wherein the composition is characterized by a release rate of the calcium alpha-ketoglutarate that is about 75% or more after a time between about 10 and about 11 hours in a dissolution medium as measured by a paddle apparatus with a basket rotating at 75 rpm wherein the dissolution medium is 900 mL of about 0.03 M NaCl and about 0.08 M HCl at about 37°C; about 75% or more after a time of about 10 hours in a dissolution medium as measured by a paddle apparatus with a basket rotating at 75 rpm wherein the dissolution medium is 900 mL of about 0.03 M NaCl and about 0.08 M HCl at about 37°C; about 75% or more after a time between about 9 and about 11 hours in a dissolution medium as measured by a paddle apparatus with a basket rotating at 75 rpm wherein the dissolution medium is 900 mL of about 0.03 M NaCl and about 0.08 M HCl at about 37°C; about 75% or more after a time of about 9 hours in a dissolution medium as measured by a paddle apparatus with a basket rotating at 75 rpm wherein the dissolution medium is 900 mL of about 0.03 M NaCl and about 0.08 M HCl at about 37°C; about 90% or more after a time between about 12 and about 15 hours in a dissolution medium as measured by a paddle apparatus with a basket rotating at 75 rpm wherein the dissolution medium is 900 mL of about 0.03 M NaCl and about 0.08 M HCl at about 37°C; about 90% or more after a time between about 12 and about 14 hours in a dissolution medium as measured by a paddle apparatus with a basket rotating at 75 rpm wherein the dissolution medium is 900 mL of about 0.03 M NaCl and about 0.08 M HCl at about 37°C; about 90% or more after a time of about 13 hours in a dissolution medium as measured by a paddle apparatus with a basket rotating at 75 rpm wherein the dissolution medium is 900 mL of about 0.03 M NaCl and about 0.08 M HCl at about 37°C; or is about 90% or more after a time of about 12 hours in a dissolution medium as measured by a paddle apparatus with a basket rotating at 75 rpm wherein the dissolution medium is 900 mL of about 0.03 M NaCl and about 0.08 M HCl at about 37°C;

The composition, including the unit dose or dosage form, of any one of the preceding clauses wherein the calcium alpha-ketoglutarate is calcium alpha-ketoglutarate monohydrate.

The composition, including the unit dose or dosage form, of any one of the preceding clauses comprising 525 mg of calcium alpha-ketoglutarate monohydrate.

The composition, including the unit dose or dosage form, of any one of the preceding clauses wherein the dosage unit is configured for oral administration; the dosage unit is a tablet; the dosage unit is a capsule.

A unit dose comprising 525 mg of Ca-AKG monohydrate and a controlled release matrix, isomalt, one or more vegetable waxes, stearic acid, magnesium stearate, an silica, where the controlled release matrix comprises HPMC.

The unit dose of any one of the preceding clauses further comprising a coating described herein.

Pharmacological treatment and prevention of the natural declines of age and aging-related diseases has presented challenges to the medical community in part because of the stringent characteristics required of pharmacological agents for this purpose. Aging patient populations impose unusually high burdens on pharmacological therapies to be bioavailable, nontoxic, and lacking long-term adverse effects. Prevention requires treatment of patients which may be at risk to aging-related disorders but experiencing no or mild symptoms, requiring agents that do not damage existing health. Treatment of patients with existing age-related disease requires a high requirement for nontoxicity, so as to not exacerbate existing health conditions. Moreover, treatment or prevention of aging-related disorders in general likely requires treatment with pharmacological agents for many years, thus requiring such agents to lack cumulative toxicity or long-term deleterious effects on organ systems.

Recent discoveries suggest that aging is driven by a molecular program operating in animals, and is not driven simply by accumulation of molecular damage. Studies in humans and model organisms aimed at elucidating the molecular mechanisms of aging have demonstrated the existence of broadly conserved longevity pathways. Various studies have demonstrated that dietary restrictions, fasting, or caloric restriction can increase lifespan and delay the onset of multiple age- related phenotypes in a diverse range of organisms, including the entire major model systems described herein.

The mTOR signaling pathway, in particular, has emerged as a central pro-aging pathway that is inhibited due to pro-longevity effects of dietary restriction in yeast, nematodes and fruit flies. In response to nutrient depletion, mTOR activity is reduced and this results in downstream events that have been shown to promote longevity and enhance resistance to environmental stressors via activation of changes in gene transcription and protein translation, as well as altered regulation of mTOR substrates through phosphorylation. Further, dietary restriction is known to have effect not only on mTOR, but also on aging processes such as the AMPK pathway (activation), sirtuin pathway (elevated protein levels), mitochondrial bioenergetics (activation), and the IGF-1 pathway (inhibition).

In some embodiments, Ca-AKG modulates the mTOR pathway. In some embodiments, Ca-AKG modulates the AMPK pathway. In some embodiments, Ca-AKG modulates the mTOR pathway and the AMPK pathway.

Unless otherwise defined, all technical and scientific terms used herein have the same meaning as commonly understood by one of ordinary skill in the art to which this disclosure belongs. Although methods and materials similar or

equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the singular includes the plural unless specifically stated otherwise. It must be noted that, as used in the specification and the appended claims, the singular forms "a", "an", and "the" include plural referents unless the context clearly dictates otherwise. In this application, the use of "or" means "and/or" unless stated otherwise. Furthermore, use of the term "including" as well as other forms, such as "include", "includes," and "included," is not limiting.

" alpha-ketoglutarate," "α-ketoglutarate," or "AKG" comprises derivatives of alpha-ketoglutarate (e.g., the derivatives set forth in MacKenzie, et al. (2007) Mol Cell Biol 27(9):3282-3289)), analogues of alpha-ketoglutarate (e.g., phosphonate analogues (e.g., those recited in Bunik, et al. (2005) Biochemistry 44(31):10552-61), esters of alpha-ketoglutarate (e.g., dimethyl alpha-ketoglutarate and octyl alpha-ketoglutarate), and various species specific analogues, e.g., human alpha-ketoglutarate, porcine alpha-ketoglutarate, murine alpha-ketoglutarate, bovine alpha-ketoglutarate, and the like.

As used herein, the terms "individual(s)", "subject(s)" and "patient(s)" mean any mammal. In some embodiments, the mammal is a human. In some embodiments, the mammal is a non-human. None of the terms require or are limited to situations characterized by the supervision (e.g. constant or intermittent) of a health care worker (e.g. a doctor, a registered nurse, a nurse practitioner, a physician's assistant, an orderly or a hospice worker).

As used herein, ranges and amounts can be expressed as "about" a particular value or range. About also includes the exact amount. Hence "about 5 μL" means "about 5 μL" and also "5 μL." Generally, the term "about" includes an amount that would be expected to be within experimental error.

The terms "controlled-release dosage form" and "controlled-release layer" are used interchangeably and defined as those whose drug release characteristics of time course and/or location are chosen to accomplish therapeutic or convenience objectives not offered by conventional immediate-release dosage forms. The rate of release of the active drug from a controlled-release layer or dosage form is controlled by features of the dosage form and/or in combination with physiologic or environmental conditions rather than by physiologic or environmental conditions alone. The controlled-release dosage forms are used to maintain drug plasma levels within the therapeutic window. The controlled-release dosage forms of certain embodiments attempt to deliver therapeutically effective amounts of active drug as a once-daily dose so that the ratio Cmax/Cmin in the plasma at steady state is less than the therapeutic index, and to maintain drug levels at constant effective levels to provide a therapeutic benefit over a period of time (e.g. 24-hour period). In certain embodiments controlled-release dosage forms provide a substantially constant or gradually decreasing rate of drug release so as to provide plasma levels which remain substantially invariant with time. In certain embodiments controlled-release dosage forms are designed to provide a quick increase in the plasma concentration of the drug which remains substantially constant within the therapeutic range of the drug for a period of time (e.g. 24-hour period). Alternatively, in some other embodiments controlled-release dosage forms are designed to provide a quick increase in the plasma concentration of the drug, which although might not remain constant, declines at a rate such that the plasma concentration remains within the therapeutic range for a period of time (e.g. 24-hour period).

The term "controlled-release matrix" refers to a matrix, such as a polymeric matrix that is capable of delivering a bioactive agent at a controlled rate for a period of time. Although there might be an initial burst phase, the overall release kinetics of the bioactive agent from the matrix are generally linear, such that a relatively constant supply of bioactive agent is released over the desired time period. The time period might vary from several hours to several days, depending upon the bioactive agent and its intended use. In general, it is preferable that the percentage of bioactive agent released from the controlled matrix over the treatment period be relatively high (e.g., at least about 50%, at least about 75%, at least about 90%, or at least about 95%) to avoid waste of unreleased bioactive agent. As used herein, the term "release-modifying agent" generally refers to components that individually or together form at least part of the controlled-release matrix.

The term "immediate-release" layer or dosage form refers to the release of an active agent substantially immediately upon administration. For example, immediate-release includes but not limited to contact with gastric juices and results in substantially complete dissolution within about 1 hour. Immediate-release components might also be referred to as instant release. When used in association with the dissolution profiles discussed herein, the term "immediate-release" refers to that portion of a dosage form disclosed herein which delivers active agent over a period of time less than 1 hour.

The terms "coating composition", "coat composition", "coating solution", "coat solution", "coating suspension", and "coat suspension" as used herein are used interchangeably and are defined to mean a mixture of excipients that is used to create a controlled-release coating. The coating composition is applied onto a calcium alpha-ketoglutarate core to form an intermediate coating, and the intermediate coating is cured to form the controlled-release coating.

The terms "effective amount", "pharmaceutically effective amount", or "therapeutically effective amount" refer to a nontoxic but sufficient amount of the agent to provide the desired biological, therapeutic, and/or prophylactic result. That result might be reduction and/or alleviation of the signs, symptoms, or causes of a disease, or any other desired alteration of a biological system. For example, an "effective amount" for therapeutic uses is the amount of the calcium alpha-

ketoglutarate as disclosed herein per se or a composition comprising the calcium alpha-ketoglutarate as disclosed herein required to provide a clinically significant decrease in a disease or condition. An appropriate effective amount in any individual case might be determined by one of ordinary skill in the art using routine experimentation.

The term "pharmaceutically acceptable," as used herein, refers a material, such as a carrier or diluent, which does not abrogate the biological activity or properties of the compound, and is relatively nontoxic, i.e., the material is administered to an individual without causing undesirable biological effects or interacting in a deleterious manner with any of the components of the composition in which it is contained.

The term "pharmaceutically acceptable salt" refers to a form of a therapeutically active agent that consists of a cationic form of the therapeutically active agent in combination with a suitable anion, or in alternative embodiments, an anionic form of the therapeutically active agent in combination with a suitable cation. Handbook of Pharmaceutical Salts: Properties, Selection and Use. International Union of Pure and Applied Chemistry, Wiley-VCR 2002. S.M. Berge, L.D. Bighley, D.C. Monkhouse, J. Pharm. Sci. 1977, 66, 1-19. P.H. Stahl and C. G. Wermuth, editors, Handbook of Pharmaceutical Salts: Properties, Selection and Use, Weinheim/Zurich: Wiley-VCH/VHCA, 2002. Pharmaceutical salts typically are more soluble and more rapidly soluble in stomach and intestinal juices than non-ionic species and so are useful in solid dosage forms. Furthermore, because their solubility often is a function of pH, selective dissolution in one or another part of the digestive tract is possible and this capability can be manipulated as one aspect of delayed and sustained release behaviors. Also, because the salt-forming molecule can be in equilibrium with a neutral form, passage through biological membranes can be adjusted.

As used herein, "treatment" or "treating" refers to an approach for obtaining beneficial or desired results with respect to a disease, disorder, or medical condition including but not limited to a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. In certain embodiments, for prophylactic benefit, the compositions are administered to a subject at risk of developing a particular disease, or to a subject reporting one or more of the physiological symptoms of a disease, even though a diagnosis of this disease may not have been made.

A "therapeutic effect," as that term is used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

"Pharmaceutically acceptable carrier, diluent or excipient" includes without limitation any adjuvant, carrier, excipient, glidant, sweetening agent, diluent, preservative, dye, colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

Alpha-Ketoglutarate (AKG)

Described herein are sustained-release compositions of alpha-ketoglutarate.

In certain aspects, the disclosure provides compositions that comprise compounds (e.g. alpha-ketoglutarate salt) that are available for human consumption without FDA approval or generally recognized as safe (GRAS). Such compounds may be so classified because they are: a) present in the FDA SCOGS database and are generally recognized as safe by the U.S. Food and Drug Administration; or b) are derived from plants (for e.g. fruits, vegetables, herbs) present in traditional diets and so are recognized by the scientific community as safe for consumption. In some embodiments GRAS compounds are those compounds which are available for human consumption without FDA approval.

In some embodiments, the compositions disclosed herein comprise AKG. Alpha-ketoglutarate or $\alpha$-ketoglutarate (Formula 1) is also known as 2-oxopentanedioic acid, 2-ketoglutaric acid, 2-oxoglutaric acid, and oxoglutaric acid. At physiological pH, $\alpha$-ketoglutarate exists in one or more deprotonated forms, such as those depicted as Formulae 2. A-ketoglutarate is an intermediate in the Krebs cycle of eukaryotic organisms and is biosynthesized from isocitrate (in the Krebs cycle process) or L-glutamate (via alanine transaminase) in such organisms. Both $\alpha$-ketoglutarate and its corresponding salts are commercially available, either via preparation from fermentation cultures (for example see US 2,776,926) or chemical synthesis from closely related compounds.

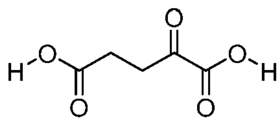

Formula 1

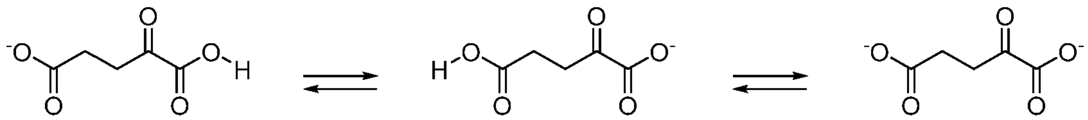

Formulae 2

Consistent with its role in energy generation via the Krebs cycle, α-ketoglutarate is an important regulator of bioenergetics in cells and is implicated as an inhibitor of ATP synthase subunit β and an indirect inhibitor of the kinase mTOR, a consequence of partial inhibition of the mitochondrial electron transport chain.

In some embodiments, α-ketoglutarate is provided as the free acid (a-ketoglutaric acid). In some embodiments, α-ketoglutarate is provided as a mono salt or bis salt. **In** other embodiments, α-ketoglutarate is provided as a monosodium salt, a disodium salt, a monopotassium salt, or a dipotassium salt. In yet further embodiments, α-ketoglutarate is provided as a mono- or di-valent salt with other cations described in the U.S. FDA *Orange Book.* Such cations include calcium, diolamine, lithium, lysine, magnesium, meglumine, olamine, tromethamine, and zinc. In further embodiments, salts of α-ketoglutarate are provided as anhydrous salts, hemihydrates, monohydrates, or dihydrates.

Further disclosed herein, in certain aspects, are compositions that comprise α-ketoglutarate salt. In some embodiments, α-ketoglutarate is provided as a calcium salt (Ca-AKG). In some embodiments, calcium α-ketoglutarate can be a hydrate calcium α-ketoglutarate. In some embodiments, calcium α-ketoglutarate can be a mono-hydrate calcium α-ketoglutarate. In some embodiments, calcium α-ketoglutarate can be hemi-hydrate calcium α-ketoglutarate. In some embodiments, calcium α-ketoglutarate can be anhydrous calcium α-ketoglutarate.

In some embodiments, the compositions disclosed herein comprise an ester of α-ketoglutarate. In some embodiments, the ester of α-ketoglutarate is a methyl ester of α-ketoglutarate. In some embodiments, the ester of α-ketoglutarate is a dimethyl ester of α-ketoglutarate. In some embodiments, the ester of α-ketoglutarate is an ethyl ester of α-ketoglutarate. In some embodiments, the ester of α-ketoglutarate is a diethyl ester of α-ketoglutarate.

In some embodiments, alpha-ketoglutarate is provided as a monolithium salt, a dilithium salt, a monosodium salt, a disodium salt, a monopotassium salt, a dipotassium salt, a calcium salt, or a zinc salt. In some embodiments, alpha-ketoglutarate is provided as a calcium salt. In further embodiments, the calcium salt of alpha-ketoglutarate is provided as anhydrous salt, monohydrate, or dihydrate. In yet further embodiments, alpha-ketoglutarate is provided as a mono- or di-valent salt with other cations described in the U.S. FDA Orange Book. Such cations include calcium, diolamine, lithium, lysine, magnesium, meglumine, olamine, tromethamine, and zinc.

Sustained-Release Compositions of Alpha-Ketoglutarate

In one aspect, described herein is a composition comprising a therapeutically effective amount of calcium alpha-ketoglutarate, and a controlled-release matrix; wherein the composition is characterized by, capable of, or configured to provide a release rate of the calcium alpha-ketoglutarate of no more than 90% of a release rate of calcium alpha-ketoglutarate of one or more calcium alpha- ketoglutarate compositions that are formulated without any controlled-release matrix over or during a predetermined period of time.

In some embodiments, the composition provides a release rate of the calcium alpha-ketoglutarate of no more than 80% of a release rate of the calcium alpha-ketoglutarate of a calcium alpha-ketoglutarate composition that is formulated without any controlled-release matrix. In some embodiments, the composition provides a release rate of the calcium alpha-ketoglutarate of no more than 70% of a release rate of the calcium alpha-ketoglutarate of a calcium alpha-ketoglutarate composition that is formulated without any controlled-release matrix. In some embodiments, the composition provides a release rate of the calcium alpha-ketoglutarate of no more than 60% of a release rate of the calcium alpha-ketoglutarate of a calcium alpha-ketoglutarate composition that is formulated without any controlled-release matrix. In some embodiments, the composition provides a release rate of the calcium alpha-ketoglutarate of no more than 50% of a release rate of the calcium alpha-ketoglutarate of a calcium alpha-ketoglutarate composition that is formulated without any controlled-release matrix. In some embodiments, the composition provides a release rate of the calcium alpha-ketoglutarate of no more than 40% of a release rate of the calcium alpha-ketoglutarate of a calcium alpha-ketoglutarate composition that is formulated without any controlled-release matrix. In some embodiments, the composition provides a release rate of the calcium alpha-ketoglutarate of no more than 30% of a release rate of the calcium alpha- ketoglutarate of a calcium alpha-ketoglutarate composition that is formulated without any controlled-release matrix. In some embodiments, the composition provides a release rate of the calcium alpha-ketoglutarate of no more than 20% of a release rate of the calcium alpha- ketoglutarate of a calcium alpha-ketoglutarate composition that is formulated without any controlled-release matrix. In some embodiments, the composition provides a release rate of the calcium alpha-ketoglutarate of no more than 10% of a release rate of the calcium alpha- ketoglutarate of a calcium alpha-ketoglutarate composition that is formulated without any controlled-release matrix. In some embodiments, the composition provides a release rate of the

calcium alpha-ketoglutarate of no more than 5% of a release rate of the calcium alpha-ketoglutarate of a calcium alpha-ketoglutarate composition that is formulated without any controlled-release matrix.

Amount of calcium alpha-ketoglutarate

In some embodiments, the amount of calcium alpha-ketoglutarate is from 15% to 85% by total weight of composition. In some embodiments, the amount of calcium alpha-ketoglutarate is from 15% to 75% by total weight of composition. In some embodiments, the amount of calcium alpha-ketoglutarate is from 30% to 70% by total weight of composition. In some embodiments, the amount of calcium alpha-ketoglutarate is from 40% to 70% by total weight of composition. In some embodiments, the amount of calcium alpha-ketoglutarate is from 30% to 65% by total weight of composition. In some embodiments, the amount of calcium alpha-ketoglutarate is from 40% to 65% by total weight of composition. In some embodiments, the amount of calcium alpha-ketoglutarate is from 30% to 60% by total weight of composition. In some embodiments, the amount of calcium alpha-ketoglutarate is from 40% to 60% by total weight of composition. In some embodiments, the amount of calcium alpha-ketoglutarate is from 45% to 55% by total weight of composition. In some embodiments, the amount of calcium alpha-ketoglutarate is from 45% to 52% by total weight of composition. In some embodiments, the amount of calcium alpha-ketoglutarate is from 46% to 53% by total weight of composition.

In some embodiments, the amount of the calcium alpha-ketoglutarate in the composition corresponds to about 10% w/w to about 75% w/w by total weight of composition. In other embodiments, the amount of the calcium alpha-ketoglutarate correspond to about 10% w/w, about 15% w/w, about 18% w/w, about 20% w/w, about 21% w/w, about 22% w/w, about 23% w/w, about 24% w/w, about 25% w/w, about 26% w/w, about 27% w/w, about 28% w/w, about 29% w/w, about 30% w/w, about 31% w/w, about 32% w/w, about 33% w/w, about 34% w/w, about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 59% w/w, about 60% w/w, about 61% w/w, about 62% w/w, about 63 w/w, about 64% w/w, about 65% w/w, about 66% w/w, about 67% w/w, about 68% w/w, about 69% w/w, about 70% w/w, about 71% w/w, about 72% w/w, about 73% w/w, about 74% w/w, or about 75% w/w by total weight of composition.

In some embodiments, the amount of calcium alpha-ketoglutarate is about 30%, 35%, 40%, 45%, 50%, 55%, 60%, or 65%, by total weight of composition. In some embodiments, the amount of calcium alpha-ketoglutarate is about 30% by total weight of composition. In some embodiments, the amount of calcium alpha-ketoglutarate is about 35% by total weight of composition. In some embodiments, the amount of calcium alpha-ketoglutarate is about 40% by total weight of composition. In some embodiments, the amount of calcium alpha-ketoglutarate is about 45% by total weight of composition. In some embodiments, the amount of calcium alpha-ketoglutarate is about 50% by total weight of composition. In some embodiments, the amount of calcium alpha- ketoglutarate is about 55% by total weight of composition. In some embodiments, the amount of calcium alpha-ketoglutarate is about 60% by total weight of composition. In some embodiments, the amount of calcium alpha-ketoglutarate is about 65% by total weight of composition.

Controlled-release matrix

In some embodiments, the controlled-release matrix comprises hydroxypropylmethylcellulose (HPMC). In some embodiments, the HPMC is selected from HPMC with number average molecular weight of between 22,000-30,000, 68,000-95,000, 115,000-150,000, 220,000-300,000, and combinations thereof. In some embodiments, the controlled-release matrix comprises HPMC with number average molecular weight of between 68,000-95,000. In some embodiments, the controlled-release matrix comprises HPMC with number average molecular weight of between 115,000-150,000. In some embodiments, the controlled-release matrix comprises HPMC with number average molecular weight of between 65,000 to 102,000 and 103,000 to 156,000. In some embodiments, the controlled-release matrix comprises HPMC with number average molecular weight of between 68,000-95,000 and 115,000- 150,000. In some embodiments, the controlled-release matrix comprises HPMC with number average molecular weight of between 77,000 to 95,000 and 109,000 to 128,000. The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

In some embodiments, the controlled-release matrix comprises HPMC with number average molecular weight of between 18,000 to 44,000. In some embodiments, the controlled-release matrix comprises HPMC with number average molecular weight of at least 18,000. In some embodiments, the controlled-release matrix comprises HPMC with number

average molecular weight of at most 44,000. In some embodiments, the controlled-release matrix comprises HPMC with number average molecular weight of between 18,000 to 20,000, 18,000 to 23,000, 18,000 to 27,000, 18,000 to 30,000, 18,000 to 33,000, 18,000 to 36,000, 18,000 to 39,000, 18,000 to 41,000, 18,000 to 44,000, 20,000 to 23,000, 20,000 to 27,000, 20,000 to 30,000, 20,000 to 33,000, 20,000 to 36,000, 20,000 to 39,000, 20,000 to 41,000, 20,000 to 44,000, 23,000 to 27,000, 23,000 to 30,000, 23,000 to 33,000, 23,000 to 36,000, 23,000 to 39,000, 23,000 to 41,000, 23,000 to 44,000, 27,000 to 30,000, 27,000 to 33,000, 27,000 to 36,000, 27,000 to 39,000, 27,000 to 41,000, 27,000 to 44,000, 30,000 to 33,000, 30,000 to 36,000, 30,000 to 39,000, 30,000 to 41,000, 30,000 to 44,000, 33,000 to 36,000, 33,000 to 39,000, 33,000 to 41,000, 33,000 to 44,000, 36,000 to 39,000, 36,000 to 41,000, 36,000 to 44,000, 39,000 to 41,000, 39,000 to 44,000, or 41,000 to 44,000. In some embodiments, the controlled- release matrix comprises HPMC with number average molecular weight of 18,000, 20,000, 23,000, 27,000, 30,000, 33,000, 36,000, 39,000, 41,000, or 44,000. The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

In some embodiments, the controlled-release matrix comprises HPMC with number average molecular weight of between 65,000 to 102,000. In some embodiments, the controlled-release matrix comprises HPMC with number average molecular weight of between at least 65,000. In some embodiments, the controlled-release matrix comprises HPMC with number average molecular weight of between at most 102,000. In some embodiments, the controlled-release matrix comprises HPMC with number average molecular weight of between 65,000 to 68,000, 65,000 to 71,000, 65,000 to 74,000, 65,000 to 77,000, 65,000 to 80,000, 65,000 to 83,000, 65,000 to 87,000, 65,000 to 91,000, 65,000 to 95,000, 65,000 to 99,000, 65,000 to 102,000, 68,000 to 71,000, 68,000 to 74,000, 68,000 to 77,000, 68,000 to 80,000, 68,000 to 83,000, 68,000 to 87,000, 68,000 to 91,000, 68,000 to 95,000, 68,000 to 99,000, 68,000 to 102,000, 71,000 to 74,000, 71,000 to 77,000, 71,000 to 80,000, 71,000 to 83,000, 71,000 to 87,000, 71,000 to 91,000, 71,000 to 95,000, 71,000 to 99,000, 71,000 to 102,000, 74,000 to 77,000, 74,000 to 80,000, 74,000 to 83,000, 74,000 to 87,000, 74,000 to 91,000, 74,000 to 95,000, 74,000 to 99,000, 74,000 to 102,000, 77,000 to 80,000, 77,000 to 83,000, 77,000 to 87,000, 77,000 to 91,000, 77,000 to 95,000, 77,000 to 99,000, 77,000 to 102,000, 80,000 to 83,000, 80,000 to 87,000, 80,000 to 91,000, 80,000 to 95,000, 80,000 to 99,000, 80,000 to 102,000, 83,000 to 87,000, 83,000 to 91,000, 83,000 to 95,000, 83,000 to 99,000, 83,000 to 102,000, 87,000 to 91,000, 87,000 to 95,000, 87,000 to 99,000, 87,000 to 102,000, 91,000 to 95,000, 91,000 to 99,000, 91,000 to 102,000, 95,000 to 99,000, 95,000 to 102,000, or 99,000 to 102,000. In some embodiments, the controlled-release matrix comprises HPMC with number average molecular weight of between 65,000, 68,000, 71,000, 74,000, 77,000, 80,000, 83,000, 87,000, 91,000, 95,000, 99,000, or 102,000.

The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

In some embodiments, the controlled-release matrix comprises HPMC with number average molecular weight of between 103,000 to 156,000. In some embodiments, the controlled-release matrix comprises HPMC with number average molecular weight of at least 103,000. In some embodiments, the controlled-release matrix comprises HPMC with number average molecular weight of at most 156,000. In some embodiments, the controlled-release matrix comprises HPMC with number average molecular weight of between 103,000 to 109,000, 103,000 to 115,000, 103,000 to 118,000, 103,000 to 123,000, 103,000 to 128,000, 103,000 to 133,000, 103,000 to 138,000, 103,000 to 142,000, 103,000 to 146,000, 103,000 to 150,000, 103,000 to 156,000, 109,000 to 115,000, 109,000 to 118,000, 109,000 to 123,000, 109,000 to 128,000, 109,000 to 133,000, 109,000 to 138,000, 109,000 to 142,000, 109,000 to 146,000, 109,000 to 150,000, 109,000 to 156,000, 115,000 to 118,000, 115,000 to 123,000, 115,000 to 128,000, 115,000 to 133,000, 115,000 to 138,000, 115,000 to 142,000, 115,000 to 146,000, 115,000 to 150,000, 115,000 to 156,000, 118,000 to 123,000, 118,000 to 128,000, 118,000 to 133,000, 118,000 to 138,000, 118,000 to 142,000, 118,000 to 146,000, 118,000 to 150,000, 118,000 to 156,000, 123,000 to 128,000, 123,000 to 133,000, 123,000 to 138,000, 123,000 to 142,000, 123,000 to 146,000, 123,000 to 150,000, 123,000 to 156,000, 128,000 to 133,000, 128,000 to 138,000, 128,000 to 142,000, 128,000 to 146,000, 128,000 to 150,000, 128,000 to 156,000, 133,000 to 138,000, 133,000 to 142,000, 133,000 to 146,000, 133,000 to 150,000, 133,000 to 156,000, 138,000 to 142,000, 138,000 to 146,000, 138,000 to 150,000, 138,000 to 156,000, 142,000 to 146,000, 142,000 to 150,000, 142,000 to 156,000, 146,000 to 150,000, 146,000 to 156,000, or 150,000 to 156,000. In some embodiments, the controlled-release matrix comprises HPMC with number average molecular weight of between 103,000, 109,000, 115,000, 118,000, 123,000, 128,000, 133,000, 138,000, 142,000, 146,000, 150,000, or 156,000. The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises

a first HPMC with number average molecular weight between about 68,000-95,000 and a second HPMC with number average molecular weight between about 115,000-150,000.

In some embodiments, the controlled-release matrix comprises HPMC with number average molecular weight of between 170,000 to 320,000. In some embodiments, the controlled-release matrix comprises HPMC with number average molecular weight of between at least 170,000. In some embodiments, the controlled-release matrix comprises HPMC with number average molecular weight of between at most 320,000. In some embodiments, the controlled-release matrix comprises HPMC with number average molecular weight of between 170,000 to 180,000, 170,000 to 190,000, 170,000 to 200,000, 170,000 to 210,000, 170,000 to 220,000, 170,000 to 230,000, 170,000 to 240,000, 170,000 to 260,000, 170,000 to 280,000, 170,000 to 300,000, 170,000 to 320,000, 180,000 to 190,000, 180,000 to 200,000, 180,000 to 210,000, 180,000 to 220,000, 180,000 to 230,000, 180,000 to 240,000, 180,000 to 260,000, 180,000 to 280,000, 180,000 to 300,000, 180,000 to 320,000, 190,000 to 200,000, 190,000 to 210,000, 190,000 to 220,000, 190,000 to 230,000, 190,000 to 240,000, 190,000 to 260,000, 190,000 to 280,000, 190,000 to 300,000, 190,000 to 320,000, 200,000 to 210,000, 200,000 to 220,000, 200,000 to 230,000, 200,000 to 240,000, 200,000 to 260,000, 200,000 to 280,000, 200,000 to 300,000, 200,000 to 320,000, 210,000 to 220,000, 210,000 to 230,000, 210,000 to 240,000, 210,000 to 260,000, 210,000 to 280,000, 210,000 to 300,000, 210,000 to 320,000, 220,000 to 230,000, 220,000 to 240,000, 220,000 to 260,000, 220,000 to 280,000, 220,000 to 300,000, 220,000 to 320,000, 230,000 to 240,000, 230,000 to 260,000, 230,000 to 280,000, 230,000 to 300,000, 230,000 to 320,000, 240,000 to 260,000, 240,000 to 280,000, 240,000 to 300,000, 240,000 to 320,000, 260,000 to 280,000, 260,000 to 300,000, 260,000 to 320,000, 280,000 to 300,000, 280,000 to 320,000, or 300,000 to 320,000. In some embodiments, the controlled-release matrix comprises HPMC with number average molecular weight of between 170,000, 180,000, 190,000, 200,000, 210,000, 220,000, 230,000, 240,000, 260,000, 280,000, 300,000, or 320,000. The claims demand that the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

Release rates

In some embodiments, the composition provides a release rate of the calcium alpha-ketoglutarate of no more than 30% at a time between 0.5 and 2 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 25% at a time between 0.5 and 2 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 25% at a time between 0.5 and 1.5 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 20% at a time between 0.5 and 1.5 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 30% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 25% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 20% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 19% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 18% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 17% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 16% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 15% at 1 hour in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at a time between 4 and 8 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at a time between 4 and 6 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at 8 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at 7 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at 6 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 60% at 5 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 55% at 5 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 50% at 5 hours in a dissolution medium as measured by a paddle ketoglutarate of no more than 45% at 5 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 50% at 4 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 45% at 4 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no more than 40% at 4 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at a time between 9 and 11 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at a time between 9 and 10 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at a time between 10 and 11 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at 9 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at 10 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 75% at 11 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 11 and 15 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 11 and 14 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 11 and 13 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 11 and 12 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 12 and 15 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 12 and 14 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at a time between 12 and 13 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at 11 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at 12 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at 13 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition provides a release rate of the calcium alpha-ketoglutarate of no less than 90% at 14 hours in a dissolution medium as measured by a paddle apparatus with a basket rotating at 75 rpm, wherein the dissolution medium is 900 mL of 0.03M NaCl and 0.08M of HCl at about 37°C.

In some embodiments, the composition comprises one or more excipients.

In some embodiments, the excipient is selected from the group consisting of diluents, fillers, disintegrants, lubricants, binders, suspending agents, and combinations thereof.

In some embodiments, the excipient is selected from the group consisting of diluents, fillers, disintegrants, lubricants, and combinations thereof.

In some embodiments, the composition comprises a first diluent. In some embodiments, the amount of the first diluent is from 11 % to 29 % by total weight of composition. In some embodiments, the amount of the first diluent is at least 11 % by total weight of composition. In some embodiments, the amount of the first diluent is at most 29 % by total weight of composition. In some embodiments, the amount of the first diluent is from 11 % to 14 %, 11 % to 17 %, 11 % to 19 %, 11 % to 20 %, 11 % to 21 %, 11 % to 23 %, 11 % to 25 %, 11 % to 27 %, 11 % to 29 %, 14 % to 17 %, 14 % to 19 %, 14 % to 20 %, 14 % to 21 %, 14 % to 23 %, 14 % to 25 %, 14 % to 27 %, 14 % to 29 %, 17 % to 19 %, 17 % to 20 %, 17 % to 21 %, 17 % to 23 %, 17 % to 25 %, 17 % to 27 %, 17 % to 29 %, 19 % to 20 %, 19 % to 21 %, 19 % to 23 %, 19 % to 25 %, 19 % to 27 %, 19 % to 29 %, 20 % to 21 %, 20 % to 23 %, 20 % to 25 %, 20 % to 27 %, 20 % to 29 %, 21 % to 23 %, 21 % to 25 %, 21 % to 27 %, 21 % to 29 %, 23 % to 25 %, 23 % to 27 %, 23 % to 29 %, 25 % to 27 %, 25 % to 29 %, or 27 % to 29 % by total weight of composition. In some embodiments, the amount of the first diluent is about 11 %, about 14 %, about 17 %, about 19 %, about 20 %, about 21 %, about 23 %, about 25 %, about 27 %, or about 29 % by total weight of composition.

In some embodiments, the first diluent is microcrystalline cellulose. In some embodiments, the amount of the microcrystalline cellulose is from 11 % to 29 % by total weight of composition. In some embodiments, the amount of the microcrystalline cellulose is from 14% to 27% by total weight of composition. In some embodiments, the amount of the microcrystalline cellulose is from 17% to 23% by total weight of composition. In some embodiments, the amount of the microcrystalline cellulose is from 17% to 22% by total weight of composition. In some embodiments, the amount of the microcrystalline cellulose is from 17% to 20% by total weight of composition. In some embodiments, the amount of the microcrystalline cellulose is at least 11 % by total weight of composition. In some embodiments, the amount of the microcrystalline cellulose is at most 29 % by total weight of composition. In some embodiments, the amount of the microcrystalline cellulose is from 11 % to 14 %, 11 % to 17 %, 11 % to 19 %, 11 % to 20 %, 11 % to 21 %, 11 % to 23 %, 11 % to

25 %, 11 % to 27 %, 11 % to 29 %, 14 % to 17 %, 14 % to 19 %, 14 % to 20 %, 14 % to 21 %, 14 % to 23 %, 14 % to 25 %, 14 % to 27 %, 14 % to 29 %, 17 % to 19 %, 17 % to 20 %, 17 % to 21 %, 17 % to 23 %, 17 % to 25 %, 17 % to 27 %, 17 % to 29 %, 19 % to 20 %, 19 % to 21 %, 19 % to 23 %, 19 % to 25 %, 19 % to 27 %, 19 % to 29 %, 20 % to 21 %, 20 % to 23 %, 20 % to 25 %, 20 % to 27 %, 20 % to 29 %, 21 % to 23 %, 21 % to 25 %, 21 % to 27 %, 21 % to 29 %, 23 % to 25 %, 23 % to 27 %, 23 % to 29 %, 25 % to 27 %, 25 % to 29 %, or 27 % to 29 % by total weight of composition. In some embodiments, the amount of the microcrystalline cellulose is about 11 % by total weight of composition. In some embodiments, the amount of the microcrystalline cellulose is about 14 % by total weight of composition. In some embodiments, the amount of the microcrystalline cellulose is about 17 % by total weight of composition. In some embodiments, the amount of the microcrystalline cellulose is about 19 % by total weight of composition. In some embodiments, the amount of the microcrystalline cellulose is about 20 % by total weight of composition. In some embodiments, the amount of the microcrystalline cellulose is about 21 % by total weight of composition. In some embodiments, the amount of the microcrystalline cellulose is about 23 % by total weight of composition. In some embodiments, the amount of the microcrystalline cellulose is about 25 % by total weight of composition. In some embodiments, the amount of the microcrystalline cellulose is about 27 % by total weight of composition. In some embodiments, the amount of the microcrystalline cellulose is about 29 % by total weight of composition.

In some embodiments, the composition comprises a second diluent. In some embodiments, the amount of the second diluent is from 4 % to 19 % by total weight of composition. In some embodiments, the amount of the second diluent is at least 4 % by total weight of composition. In some embodiments, the amount of the lactose monohydrate is at most 19 % by total weight of composition. In some embodiments, the amount of the second diluent is from 4 % to 6 %, 4 % to 8 %, 4 % to 10 %, 4 % to 11 %, 4 % to 12 %, 4 % to 13 %, 4 % to 14 %, 4 % to 15 %, 4 % to 17 %, 4 % to 19 %, 6 % to 8 %, 6 % to 10 %, 6 % to 11 %, 6 % to 12 %, 6 % to 13 %, 6 % to 14 %, 6 % to 15 %, 6 % to 17 %, 6 % to 19 %, 8 % to 10 %, 8 % to 11 %, 8 % to 12 %, 8 % to 13 %, 8 % to 14 %, 8 % to 15 %, 8 % to 17 %, 8 % to 19 %, 10% to 11 %, 10 % to 12 %, 10 % to 13 %, 10 % to 14 %, 10 % to 15 %, 10 % to 17 %, 10 % to 19 %, 11 % to 12 %, 11 % to 13 %, 11 % to 14 %, 11 % to 15 %, 11 % to 17 %, 11 % to 19 %, 12 % to 13 %, 12 % to 14 %, 12 % to 15 %, 12 % to 17 %, 12 % to 19 %, 13 % to 14 %, 13 % to 15 %, 13 % to 17 %, 13 % to 19 %, 14 % to 15 %, 14 % to 17 %, 14 % to 19 %, 15 % to 17 %, 15 % to 19 %, or 17 % to 19 % by total weight of composition. In some embodiments, the amount of the second diluent is about 4 %, about 6 %, about 8 %, about 10 %, about 11 %, about 12 %, about 13 %, about 14 %, about 15 %, about 17 %, or about 19 % by total weight of composition.

In some embodiments, the second diluent is lactose monohydrate. In some embodiments, the amount of the lactose monohydrate is from 4 % to 19 % by total weight of composition. In some embodiments, the amount of the lactose monohydrate is from 6% to 19% by total weight of composition. In some embodiments, the amount of the lactose monohydrate is from 8% to 17% by total weight of composition. In some embodiments, the amount of the lactose monohydrate is from 10% to 15% by total weight of composition. In some embodiments, the amount of the lactose monohydrate is from 11% to 14% by total weight of composition. In some embodiments, the amount of the lactose monohydrate is at least 4 % by total weight of composition. In some embodiments, the amount of the lactose monohydrate is at most 19 % by total weight of composition. In some embodiments, the amount of the lactose monohydrate is from 4 % to 6 %, 4 % to 8 %, 4 % to 10 %, 4 % to 11 %, 4 % to 12 %, 4 % to 13 %, 4 % to 14 %, 4 % to 15 %, 4 % to 17 %,4 % to 19 %, 6 % to 8 %, 6 % to 10 %, 6 % to 11 %, 6 % to 12 %, 6 % to 13 %, 6 % to 14 %, 6 % to 15 %, 6 % to 17 %, 6 % to 19 %, 8 % to 10 %, 8 % to 11 %, 8 % to 12 %, 8 % to 13 %, 8 % to 14 %, 8 % to 15 %, 8 % to 17 %, 8 % to 19 %, 10 % to 11 %, 10 % to 12 %, 10 % to 13 %, 10 % to 14 %, 10 % to 15 %, 10 % to 17 %, 10 % to 19 %, 11 % to 12 %, 11 % to 13 %, 11 % to 14 %, 11 % to 15 %, 11 % to 17 %, 11 % to 19 %, 12 % to 13 %, 12 % to 14 %, 12 % to 15 %, 12 % to 17 %, 12 % to 19 %, 13 % to 14 %, 13 % to 15 %, 13 % to 17 %, 13 % to 19 %, 14 % to 15 %, 14 % to 17 %, 14 % to 19 %, 15 % to 17 %, 15 % to 19 %, or 17 % to 19 % by total weight of composition. In some embodiments, the amount of the lactose monohydrate is about 4 % by total weight of composition. In some embodiments, the amount of the lactose monohydrate is about 6 % by total weight of composition. In some embodiments, the amount of the lactose monohydrate is about 8 % by total weight of composition. In some embodiments, the amount of the lactose monohydrate is about 10 % by total weight of composition. In some embodiments, the amount of the lactose monohydrate is about 11 % by total weight of composition. In some embodiments, the amount of the lactose monohydrate is about 12 % by total weight of composition. In some embodiments, the amount of the lactose monohydrate is about 13 % by total weight of composition. In some embodiments, the amount of the lactose monohydrate is about 14 % by total weight of composition. In some embodiments, the amount of the lactose monohydrate is about 15 % by total weight of composition. In some embodiments, the amount of the lactose monohydrate is about 17 % by total weight of composition. In some embodiments, the amount of the lactose monohydrate is about 19 % by total weight of composition.

In some embodiments, the composition comprises a lubricant. In some embodiments, the amount of the lubricant is from 1 % to 5 % by total weight of composition. In some embodiments, the amount of the lubricant is at least 1 % by total weight of composition. In some embodiments, the amount of the lubricant is at most 5 % by total weight of composition. In some embodiments, the amount of the lubricant is from 1 % to 2 %, 1 % to 3 %, 1 % to 4 %, 1 % to 5 %, 2 % to 3 %, 2 % to 4 %, 2 % to 5 %, 3 % to 4 %, 3 % to 5 %, or 4 % to 5 % by total weight of composition. In some embodiments, the amount of the lubricant is 1 %, 2 %, 3 %, 4 %, or 5 % by total weight of composition.

In some embodiments, the lubricant is magnesium stearate. In some embodiments, the amount of the

magnesium stearate is 1 % to 5 % by total weight of composition. In some embodiments, the amount of magnesium stearate is from 1% to 3% by total weight of composition. In some embodiments, the amount of magnesium stearate is from 2% to 4% by total weight of composition. In some embodiments, the amount of the magnesium stearate is at least 1 % by total weight of composition. In some embodiments, the amount of the magnesium stearate is at most 5 % by total weight of composition. In some embodiments, the amount of the magnesium stearate is 1 % to 2 %, 1 % to 3 %, 1 % to 4 %, 1 % to 5 %, 2 % to 3 %, 2 % to 4 %, 2 % to 5 %, 3 % to 4 %, 3 % to 5 %, or 4 % to 5 % by total weight of composition. In some embodiments, the amount of the magnesium stearate is 1 %, 2 %, 3 %, 4 %, or 5 % by total weight of composition.

In some embodiments, the amount of the magnesium stearate is about 1 % by total weight of composition. In some embodiments, the amount of the magnesium stearate is about 2 % by total weight of composition. In some embodiments, the amount of the magnesium stearate is about 3 % by total weight of composition. In some embodiments, the amount of the magnesium stearate is about 4 % by total weight of composition. In some embodiments, the amount of the magnesium stearate is about 5 % by total weight of composition.

In some embodiments, the composition comprises a disintegrant. In some embodiments, the amount of the disintegrant is from 1 % to 5 % by total weight of composition. In some embodiments, the amount of the disintegrant is at least 1 % by total weight of composition. In some embodiments, the amount of the disintegrant is at most 5 % by total weight of composition. In some embodiments, the amount of the disintegrant is from 1 % to 2 %, 1 % to 3 %, 1 % to 4 %, 1 % to 5 %, 2 % to 3 %, 2 % to 4 %, 2 % to 5 %, 3 % to 4 %, 3 % to 5 %, or 4 % to 5 % by total weight of composition. In some embodiments, the amount of the disintegrant is 1 %, 2 %, 3 %, 4 %, or 5 % by total weight of composition.

In some embodiments, the disintegrant is silicon dioxide. In some embodiments, the amount of the silicon dioxide is from 1 % to 5 % by total weight of composition. In some embodiments, the amount of silicon dioxide is from 1 % to 3% by total weight of composition. In some embodiments, the amount of silicon dioxide is from 2% to 4% by total weight of composition. In some embodiments, the amount of the silicon dioxide is at least 1 % by total weight of composition. In some embodiments, the amount of the silicon dioxide is at most 5 % by total weight of composition. In some embodiments, the amount of the silicon dioxide is from 1 % to 2 %, 1 % to 3 %, 1 % to 4 %, 1 % to 5 %, 2 % to 3 %, 2 % to 4 %, 2 % to 5 %, 3 % to 4 %, 3 % to 5 %, or 4 % to 5 % by total weight of composition.

In some embodiments, the amount of the silicon dioxide is about 1 % by total weight of composition. In some embodiments, the amount of the silicon dioxide is about 2 % by total weight of composition. In some embodiments, the amount of the silicon dioxide is about 3 % by total weight of composition. In some embodiments, the amount of the silicon dioxide is about 4 % by total weight of composition. In some embodiments, the amount of the silicon dioxide is about 5 % by total weight of composition.

## Compositions of Alpha-Ketoglutarate

In another aspect, described herein is a composition comprising: calcium alpha-ketoglutarate, and one or more agents selected from the group consisting of surfactants, preservatives, flavoring agents, vitamins, antioxidants, sweetening agents, and combinations thereof.

In some embodiments, the amount of calcium alpha-ketoglutarate is from 50 mg to 5000 mg. In some embodiments, the amount of calcium alpha-ketoglutarate is from 100 mg to 2000 mg. In some embodiments, the amount of calcium alpha-ketoglutarate is about 250 mg. In some embodiments, the amount of calcium alpha-ketoglutarate is about 500 mg. In some embodiments, the amount of calcium alpha-ketoglutarate is about 525 mg. In some embodiments, the amount of calcium alpha-ketoglutarate is about 550 mg. In some embodiments, the amount of calcium alpha-ketoglutarate is about 750 mg.

In some embodiments, the calcium alpha-ketoglutarate is calcium alpha-ketoglutarate monohydrate.

In some embodiments, the composition comprises a vitamin. In some embodiments, the vitamin is vitamin A. In some embodiments, the amount of vitamin A is from 100 mcg to 3000 mcg; from 200 mcg to 1000 mcg; about 250 mcg; about 450 mcg; or about 650 mcg. In some embodiments, the vitamin A is retinyl palmitate.

In some embodiments, the vitamin is vitamin D. In some embodiments, the amount of vitamin D is from 50 IU to 3000 IU; from 200 IU to 2000 IU; or about 250 IU. In some embodiments, the amount of vitamin D is about 500 IU. In some embodiments, the amount of vitamin D is about 750 IU. In some embodiments, the vitamin D is cholecalciferol.

In some embodiments, the composition further comprises a sweetener. In some embodiments, the sweetener is isomalt.

In some embodiments, the composition further comprises wax. In some embodiments, the wax is carnauba wax and/or rice bran wax.

In some embodiments, the composition further comprises one or more excipients. In some embodiments, the excipient is selected from the group consisting of solubilizing agents, diluents, fillers, disintegrants, lubricants, glidants, binders, suspending agents, and combinations thereof. In some embodiments, the excipient is selected from the group consisting of solubilizing agents, diluents, fillers, disintegrants, lubricants, glidants, and combinations thereof.

In some embodiments, the composition further comprises a first lubricant. In some embodiments, the first

lubricant is stearic acid. In some embodiments, the composition comprises a second lubricant. In some embodiments, the second lubricant is magnesium stearate. In some embodiments, the composition comprises a glidant. In some embodiments, the glidant is silica.

In another aspect, also described herein is a composition comprising 500 mg of calcium alpha-ketoglutarate monohydrate; 450 mcg of retinyl palmitate; and further comprising isomalt, vegetable wax (carnauba and/or rice bran), stearic acid, magnesium stearate, and silica.

In another aspect, also described herein is a composition comprising 500 mg of calcium alpha-ketoglutarate monohydrate; 12.5 mcg (500 IU) of cholecalciferol; and further comprising isomalt, vegetable wax (carnauba and/or rice bran), stearic acid, magnesium stearate, and silica.

Controlled-Release Matrix Formulations

In certain embodiments, the composition of the calcium alpha-ketoglutarate described herein comprises a controlled-release matrix. There are many mechanisms by which a bioactive agent might be released from a controlled-release matrix. Two mechanisms include diffusion and/or degradation. Diffusion occurs when the bioactive agent is released either through pores in the polymer matrix or by passing between polymer chains of the matrix. In a diffusion system, the bioactive agent might be dispersed throughout the matrix, or localized within a reservoir adjacent to or within the matrix. In a reservoir system, a reservoir of bioactive agent, for example, solid drug, dilute solution, or highly concentrated drug solution within a polymer matrix is surrounded by a controlled-release material through which the bioactive agent is able to diffuse. In a degradable system, the bioactive agent is released as the matrix is degraded in vivo. Bioactive agent might also be released by a combination of the two mechanisms. In some embodiment of the controlled-release matrix described herein, the release of the bioactive agent is driven by a combination of both diffusion and degradation. The release rate might be controlled by varying the drug to polymer ratio (e.g., a higher drug concentration tends to result in a faster rate of release), by varying the chemistry of polymeric matrix (e.g., inclusion of polymers having a Tg of less than about 40° C or less than about 0° C would tend to result in a faster elution rate than polymers with Tgs greater than 40° C, polymers that absorb water tend to elute drug more quickly than more hydrophobic polymers that do not absorb water. These variables might be controlled by the selection of materials used in the manufacturing process.

In some embodiments, the controlled-release matrix is configured to release the bioactive agent as set forth above. In some embodiments, the controlled-release matrix is configured to release at least about 40% and up to about 60%, or at least 50% of the bioactive agent within 12 hours of administration. In some embodiments, the controlled-release matrix is configured to release at least about 40% and up to about 60%, or at least 50% of the bioactive agent within 24 hours of administration. In another embodiment, the controlled-release matrix is configured to release at least about 80% or up to about 100%, or at least 90% of the bioactive agent within 7 days after administration.

In some embodiments, the controlled-release matrix is biodegradable. In some embodiments, the controlled-release matrix includes a biodegradable polyester. Examples of biodegradable polyesters include, but are not limited to: polycaprolactone (PCL), polylactic acid (PLA), polyglycolide (PGA), and copolymers thereof, such as poly(lactic-co-glycolic acid) polymers (PLGA) and poly(glycolide-co-caprolactone) (PGC). Polycaprolactone (PCL) refers to a biodegradable polyester prepared by ring opening polymerization of ε-caprolactone using a catalyst such as stannous octanoate. Polycaprolactone has a melting point of about 60° C and is degraded by hydrolysis of its ester linkages under physiological conditions.

Polylactic acid (PLA) is a biodegradable, thermoplastic polyester that can be produced by bacterial fermentation of renewable resources such as corn, starch or sugarcane and has a melting temperature between about 173° C and about 178° C.

Polyglycolide (PGA) is a biodegradable, thermoplastic polyester prepared from glycolic acid by polycondensation or ring-opening polymerization. It has a melting point of between about 225° C to about 230° C.

Poly(lactic-co-glycolic acid) polymers (PLGA) refers to a biodegradable copolymer of lactic and glycolic acid formed by random ring-opening co-polymerization of monomers of glycolic acid and lactic acid. During polymerization, the monomeric units are linked together by ester linkages, thus yielding an aliphatic polyester. PLGAs are amorphous and have a glass transition temperature between about 40° C and 60° C. In general, the PLGA copolymer has a weight average molecular weight between about 1000 Dato about 50,000 Da, or between about 5000 Da and 25,000 Da. The ratio of lactic acid to glycolic acid might vary. In general and increase in the amount of lactic acid results in a polymer that degrades more slowly. An increase in glycolic acid results in a polymer that degrades more quickly. Additionally, an increase in glycolic acid tends to decrease the glass transition temperature (Tg) and water penetration into the polymer, which can result in a faster release of compounds. In general, the ratio of lactic acid to glycolic acid is between about 100:0 to about 25:75, or between about 60:40 and 40:60, or about 50:50.

Other suitable biodegradable polymers include, but are not limited to, poly(trimethylene carbonate) (PTMC), polydioxanone (PDO), poly(4-hydroxy butyrate) (PHB), and poly(butylene succinate) (PBS), poly(trimethylene carbonate) (PTMC), polydioxanone (PDO), poly(4-hydroxy butyrate) (PHB), and poly(butylene succinate) (PBS).

In some embodiments, the polymeric material or polymer is biostable. Examples of biostable polymers include, but are not limited to polyurethanes, silicone rubbers, styrene-isobutylene-styrene block copolymers, ether-ester block copolymers (e.g., 1500-40D from RTP Co.) and vinyl materials, including but not limited to poly(ethylene-co-vinyl acetate) (PEVA).

In some embodiments, the controlled-release matrix includes an elastomeric polymeric material that includes a copolymer with an elastomeric (or "soft") component and a non- elastomeric (or "hard") component. In another embodiment, the elastomeric polymeric material includes a polymeric blend having an elastomeric component and a non-elastomeric component.

In some embodiments, the compliant polymer or polymeric material is thermoplastic. As used herein, the term "thermoplastic" refers to a polymer or polymeric material that can be softened by heat, hardened by cooling and then softened by heat over and over again. In general, thermoplastic materials are not cross-linked. However, in another embodiment, the compliant polymer or polymeric material might be cross-linked.

The bioactive agent might be incorporated into the controlled-release matrix any of various techniques known to the skilled artisan. In one embodiment, the bioactive agent is dispersed throughout the controlled-release matrix. Techniques for preparing the controlled-release matrix include, but are not limited to, melt extrusion processes, injection molding, or spray casting.

In a melt extrusion process, a mixture that includes the polymeric material and bioactive agent is combined in an extruder, heated to a temperature at which the polymeric material melts and then discharged through an orifice of the desired cross-sectional shape. The extruded material is collected under controlled conditions (e.g., speed, temperature and humidity) to obtain a product with the desired dimensions. In one embodiment, the mass flow rate of the extrudate and the collection speed of the final extruded form might be controlled to achieve the desired physical dimensions. For example, if the final extruded form is a film, then the collection speed of the film might be increased relative to the mass flow rate of the extrudate to decrease the film thickness, and conversely to increase the film thickness. The extrudate is discharged through an orifice in the molten state, allowing elongation of the extrudate to its final dimension. The extrudate is subsequently cooled by exposure to ambient conditions, a chilled liquid or gas bath, or exposure to a temperature controlled surface such as a cooled roller in order to solidify the extrudate. In one embodiment, the melt extrusion process is used to form a film. In an alternate embodiment, the melt extrusion process is used to form pellets or beads that might be subsequently molded into the desired film or collar configuration. Some of the advantages of melt extrusion processes include: the absence of organic solvents and high throughput, continuous manufacturing. In general, the processing temperature is sufficient to melt the polymeric material without adversely affecting the biological activity of the bioactive agent. In general, the processing temperature is at least about 80° C or about 100° C, and less than about 180° C, less than 160° C, or between about 110° C and about 150° C. In some embodiments, the specific temperature is dependent on the melting and degradation temperatures of the polymeric materials and bioactive agent. Furthermore, melt-processing provides the ability for continuous operation, the ability to control operating parameters, and the ability to scale up manufacturing.

In an alternate embodiment, an injection molding process is used. In an injection molding process, a mixture that includes the polymeric material and bioactive agent is fed into a vessel where it heated to a temperature sufficient to melt the polymeric material and then forced into a mold cavity where it cools and hardens to the configuration of the mold cavity. The conditions (e.g., temperature and pressure) will depend upon the material being molded. In one embodiment, the injection molding process is used to form a film or a collar.

In yet another embodiment, a solvent casting technique might be used. In a solvent casting process, the polymeric material and bioactive agent are combined with a suitable solvent to form a polymeric solution which is then cast on a substrate. The solvent is then removed to form a film, for example, by evaporation. In one embodiment, the solvent is removed under a vacuum (e.g., between about 15 in Hg and about 28 in Hg, depending upon the volatility of the solvent). In another embodiment, the solvent is removed at an elevated temperature (e.g., between about 30° C and about 80° C). In an alternate embodiment, the polymeric solution is applied to the substrate by a spray coating process. In a spray coating process, the polymeric solution is fed to the spray nozzle, for example and ultrasonic spray nozzle, at a controlled rate by a positive displacement pump. The spray nozzle and substrate are moved in relative motion to each other at controlled speed to achieve the desired coating thickness. The spray nozzle is mounted on a three-axis motion control system (x-y-z) which is capable of controlling the speed and position of the spray head relative to the substrate. In addition, if the substrate is a rolled film, it is traversed below the spray head by a roll to roll unwinding and winding apparatus. The coating width is controlled by moving the spray nozzle in a specified path across the width of the substrate. In addition, the height (z) of the spray nozzle above the substrate might be increased to achieve a wider coating width.

The solvent might be one in which one or more components of the polymeric material form a true solution. The bioactive agent might either be soluble in the solvent or form a dispersion throughout the solvent. Suitable solvents include, but are not limited to, alcohols (e.g., methanol, butanol, propanol and isopropanol), alkanes (e.g., halogenated or unhalogenated alkanes such as hexane, cyclohexane, methylene chloride and chloroform), amides (e.g., dimethylformamide), ethers (e.g., tetrahydrofuran (THF), dioxolane, and dioxane), ketones (e.g., methyl ethyl ketone, acetone),

aromatic compounds (e.g., toluene and xylene), nitriles (e.g., acetonitrile) and esters (e.g., ethyl acetate). THF and chloroform have been found to be suitable solvents due to their excellent solvency for a variety of polymers and bioactive agents.

Excipients

In certain embodiments, the composition of the calcium alpha-ketoglutarate described herein comprises an excipient. In some embodiments, the composition disclosed herein contain excipients, such as a suspending agent (e.g., methyl cellulose), a wetting agent (e.g., lecithin, lysolecithin and/or a long-chain fatty alcohol), as well as coloring agents, preservatives, flavoring agents, and the like.

Compositions disclosed herein might also be integrated into foodstuffs, e.g., cream cheese, butter, salad dressing, or ice cream to facilitate solubilization, administration, and/or compliance in certain patient populations.

Preparations for oral use might be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; flavoring elements, cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents might be added, such as the cross- linked polyvinylpyrrolidone, agar, or alginic acid or a salt thereof such as sodium alginate. The active compounds might also be formulated as a sustained release preparation.

Dragee cores might be provided with suitable coatings. For this purpose, concentrated sugar solutions might be used, which might optionally contain gum arabic, talc, polyvinylpyrrolidone, carbopol gel, polyethylene glycol, and/or titanium dioxide, lacquer solutions, and suitable organic solvents or solvent mixtures. Dyestuffs or pigments might be added to the tablets or dragee coatings for identification or to characterize different combinations of active compound doses.

Preparations that might be used orally include push-fit capsules made of gelatin, as well as soft, sealed capsules made of gelatin and a plasticizer, such as glycerol or sorbitol. The push- fit capsules might contain the active ingredients in admixture with filler such as lactose, binders such as starches, and/or lubricants such as talc or magnesium stearate and, optionally, stabilizers. In soft capsules, the active compounds might be dissolved or suspended in suitable liquids, such as fatty oils, liquid paraffin, or liquid polyethylene glycols. In addition, stabilizers might be added. All compositions for oral administration should be in dosages suitable for administration.

For injection, the compositions disclosed herein might be formulated in aqueous solutions, preferably in physiologically compatible buffers such as Hank's solution, Ringer's solution, or physiological saline buffer. Such compositions might also include one or more excipients, for example, preservatives, solubilizers, fillers, lubricants, stabilizers, albumin, and the like. Methods of formulation are known in the art, for example, as disclosed in Remington's Pharmaceutical Sciences, latest edition, Mack Publishing Co., Easton, Pa. These compositions might also be formulated for transmucosal administration, buccal administration, for administration by inhalation, for parental administration, for transdermal administration, and rectal administration.

In addition to the disclosed formulations, the compositions might also be formulated as a depot preparation. Such long acting formulations might be administered by implantation or transcutaneous delivery (for example subcutaneously or intramuscularly), intramuscular injection or use of a transdermal patch. Thus, for example, the compositions might be formulated with suitable polymeric or hydrophobic materials (for example as an emulsion in an acceptable oil) or ion exchange resins, or as sparingly soluble derivatives, for example, as a sparingly soluble salt.

In some embodiments, the formulations of the compositions disclosed herein include, but are not limited to, aqueous liquid dispersions, self-emulsifying dispersions, solid solutions, liposomal dispersions, aerosols, solid dosage forms, powders, immediate-release formulations, controlled-release formulations, fast melt formulations, tablets, capsules, pills, delayed release formulations, extended release formulations, pulsatile release formulations, multiparticulate formulations (e.g., nanoparticle formulations), and mixed immediate-and controlled-release formulations.

In some embodiments, the formulations include a carrier or carrier materials selected on the basis of compatibility with the composition disclosed herein, and the release profile properties of the desired dosage form. Compatible carrier materials include, but are not limited to, acacia, gelatin, colloidal silicon dioxide, calcium glycerophosphate, calcium lactate, maltodextrin, glycerin, magnesium silicate, polyvinylpyrrollidone (PVP), cholesterol, cholesterol esters, sodium caseinate, soy lecithin, taurocholic acid, phosphotidylcholine, sodium chloride, tricalcium phosphate, dipotassium phosphate, cellulose and cellulose conjugates, sugars sodium stearoyl lactylate, carrageenan, monoglyceride, diglyceride, pregelatinized starch, and any combination thereof. See, e.g., Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed.

(Lippincott Williams & Wilkins 1999).

In some instances, the formulations further include pH adjusting agents or buffering agents which include acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

In some instances, the formulation includes one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In some instances, the formulations include binder which are used to hold the calcium alpha-ketoglutarate and inactive ingredients together in a cohesive mix. Suitable binders include, but are not limited to, carboxymethylcellulose, methylcellulose (e.g., Methocel®), hydroxypropylmethylcellulose (e.g. Hypromellose USP Pharmacoat-603, hydroxypropylmethylcellulose acetate stearate (Aqoate HS-LF and HS), hydroxyethylcellulose, hydroxypropylcellulose (e.g., Klucel®), ethylcellulose (e.g., Ethocel®), and microcrystalline cellulose (e.g., Avicel®), microcrystalline dextrose, amylase, magnesium aluminum silicate, polysaccharide acids, bentonites, gelatin, polyvinylpyrrolidone/vinyl acetate copolymer, crospovidone, povidone, starch, pregelatinized starch, tragacanth, dextrin, a sugar, such as sucrose (e.g., Dipac®), glucose, dextrose, molasses, mannitol, sorbitol, xylitol (e.g., Xylitab®), lactose, a natural or synthetic gum such as acacia, tragacanth, ghatti gum, mucilage of isapol husks, starch, polyvinylpyrrolidone (e.g., Povidone® CL, Kollidon® CL, Polyplasdone® XL-10, and Povidone® K-12), larch arabogalactan, Veegum®, polyethylene glycol, waxes, sodium alginate, and any combination thereof.

In some instances, the formulations further include diluent which are used to stabilize the calcium alpha-ketoglutarate because they provide a more stable environment. Salts dissolved in buffered solutions (which also provide pH control or maintenance) are utilized as diluents in the art, including, but not limited to a phosphate buffered saline solution. In certain instances, diluents increase bulk of the composition to facilitate compression or create sufficient bulk for homogenous blend for capsule filling. Such compounds include e.g., lactose, starch, mannitol, sorbitol, dextrose, microcrystalline cellulose such as Avicel®; dibasic calcium phosphate, dicalcium phosphate dihydrate; tricalcium phosphate, calcium phosphate; anhydrous lactose, spray-dried lactose; pregelatinized starch, compressible sugar, such as Di-Pac® (Amstar); mannitol, hydroxypropylmethylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylase; powdered cellulose, calcium carbonate; glycine, kaolin; mannitol, sodium chloride; inositol, bentonite, and any combination thereof.

In some cases, the formulations include disintegration agents or disintegrants to facilitate the breakup or disintegration of a substance. The term "disintegrate" include both the dissolution and dispersion of the dosage form when contacted with gastrointestinal fluid. Examples of disintegration agents include a starch, e.g., a natural starch such as corn starch or potato starch, a pregelatinized starch such as National 1551 or Amijel®, or sodium starch glycolate such as Promogel® or Explotab®, a cellulose such as a wood product, methylcrystalline cellulose, e.g., Avicel®, Avicel® PHIOI, Avicel®PH102, Avicel® PH105, Elcema® PI 00, Emcocel®, Vivacel®, Ming Tia®, and Solka-Floc®, methylcellulose, croscarmellose, or a cross-linked cellulose, such as cross-linked sodium carboxymethylcellulose (Ac-Di-Sol®), cross-linked carboxymethylcellulose, or cross-linked croscarmellose, a cross-linked starch such as sodium starch glycolate, a cross-linked polymer such as crospovidone, a cross-linked polyvinylpyrrolidone, alginate such as alginic acid or a salt of alginic acid such as sodium alginate, a clay such as Veegum® HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and any combination thereof.

In some instances, the formulations include filling agents such as lactose, calcium carbonate, calcium phosphate, dibasic calcium phosphate, calcium sulfate, microcrystalline cellulose, cellulose powder, dextrose, dextrates, dextran, starches, pregelatinized starch, hydroxypropylmethylcellulose (HPMC), hydroxypropylmethylcellulose phthalate, hydroxypropylmethylcellulose acetate stearate (HPMCAS), sucrose, xylitol, lactitol, mannitol, sorbitol, sodium chloride, polyethylene glycol, and any combination thereof.

Lubricants and glidants are also optionally included in the formulations disclosed herein for preventing, reducing or inhibiting adhesion or friction of materials. Exemplary lubricants include, e.g., stearic acid, calcium hydroxide, talc, sodium stearyl fumerate, a hydrocarbon such as mineral oil, or hydrogenated vegetable oil such as hydrogenated soybean oil (Sterotex®), higher fatty acids and their alkali-metal and alkaline earth metal salts, such as aluminum, calcium, magnesium, zinc, stearic acid, sodium stearates, glycerol, talc, waxes, Stearowet®, boric acid, sodium benzoate, sodium acetate, sodium chloride, leucine, a polyethylene glycol (e.g., PEG-4000) or a methoxypolyethylene glycol such as Carbowax™, sodium oleate, sodium benzoate, glyceryl behenate, polyethylene glycol, magnesium or sodium lauryl sulfate, colloidal silica such as Syloid™, Cab-O-Sil ®, a starch such as com starch, silicone oil, a surfactant, and any

combination thereof.

Plasticizers include compounds used to soften the microencapsulation material or film coatings to make them less brittle. Suitable plasticizers include, e.g., polyethylene glycols such as PEG 300, PEG 400, PEG 600, PEG 1450, PEG 3350, and PEG 800, stearic acid, propylene glycol, oleic acid, triethyl cellulose and triacetin. Plasticizers also function as dispersing agents or wetting agents.

Solubilizers include compounds such as triacetin, triethylcitrate, ethyl oleate, ethyl caprylate, sodium lauryl sulfate, sodium doccusate, vitamin E TPGS, dimethylacetamide, N-methyl pyrrolidone, N-hydroxyethylpyrrolidone, polyvinylpyrrolidone, hydroxypropylmethylcellulose, hydroxypropyl cyclodextrins, ethanol, n-butanol, isopropyl alcohol, cholesterol, bile salts, polyethylene glycol 200-600, glycofurol, transcutol, propylene glycol, and dimethyl isosorbide and any combination thereof.

Stabilizers include compounds such as any antioxidation agents, buffers, acids, preservatives and any combination thereof.

Suspending agents include compounds such as polyvinylpyrrolidone, e.g., polyvinylpyrrolidone Kl 2, polyvinylpyrrolidone Kl 7, polyvinylpyrrolidone K25, or polyvinylpyrrolidone K30, vinyl pyrrolidone/vinyl acetate copolymer (S630), polyethylene glycol, e.g., the polyethylene glycol has a molecular weight of about 300 to about 6000, or about 3350 to about 4000, or about 7000 to about 5400, sodium carboxymethylcellulose, methylcellulose, hydroxypropylmethylcellulose, hydroxymethyl cellulose acetate stearate, polysorbate-80, hydroxyethyl cellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethyl cellulose, hydroxyethyl cellulose, polysorbate-80, sodium alginate, polyethoxylated sorbitan monolaurate, polyethoxylated sorbitan monolaurate, povidone, and any combination thereof.

Surfactants include compounds such as sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic® (BASF), and any combination thereof. Additional surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40. Sometimes, surfactants are included to enhance physical stability or for other purposes.

Viscosity enhancing agents include, e.g., methyl cellulose, xanthan gum, carboxymethyl cellulose, hydroxypropyl cellulose, hydroxypropylmethylcellulose, hydroxypropylmethylcellulose acetate stearate, hydroxypropylmethylcellulose phthalate, carbomer, polyvinyl alcohol, alginates, acacia, chitosans and combinations thereof.

Wetting agents include compounds such as oleic acid, glyceryl monostearate, sorbitan monooleate, sorbitan monolaurate, triethanolamine oleate, polyoxyethylene sorbitan monooleate, polyoxyethylene sorbitan monolaurate, sodium docusate, sodium oleate, sodium lauryl sulfate, sodium doccusate, triacetin, Tween 80, vitamin E TPGS, ammonium salts, and any combination thereof.

Antifoaming agents are chemical additive that reduces and hinders the formation of foam in the preparation of an oral liquid formulation. The terms antifoaming agent and defoamer are often used interchangeably. Commonly used agents are insoluble oils, polydimethylsiloxanes (e.g., simethicone) and other silicones, certain alcohols, stearates and glycols. The additive is used to prevent formation of foam or is added to break foam already formed. Antifoaming agents reduce foaming in the preparation of an oral liquid formulation which might result in coagulation of aqueous dispersions. In some embodiments, the calcium alpha-ketoglutarate compositions described herein comprise an antifoaming agent. In some embodiments, the antifoaming agent is simethicone.

In some embodiments, there is a considerable overlap between excipients used in the compositions, formulations, and dosage forms of the calcium alpha-ketoglutarate described herein. Thus, the above-listed additives should be taken as merely exemplary, and not limiting, of the types of additives that might be included in solid dosage forms of the compositions described herein.

Bi-layer formulations

In some embodiments, the composition described herein is formulated as a bi-layer formulation. In some instances, the bi-layer formulation comprising the calcium alpha-ketoglutarate has an enhanced bioavailability and has a lower administration dose. In some embodiments, the bi- layer formulation is an oral dosage form comprising an immediate-release top layer and a controlled- release core.

Controlled-Release Coating Formulations

In some embodiments, at least one controlled-release coating surrounds the core of the oral dosage form. In certain embodiments the controlled-release coating is a stable controlled- release monolithic coating that is formed by a process that comprises coating the core with a coating composition to form a coated core with an intermediate coating, and

curing the coated core to form the stable controlled-release coating. In at least one embodiment the coating composition comprises an aqueous dispersion of a neutral ester copolymer without any functional groups, a poly glycol having a melting point of at least 55° C, and one or more second excipients. The curing is conducted at a temperature at least equal to or greater than the melting point of the poly glycol. In at least one embodiment the stable controlled-release coating comprises a neutral ester copolymer without any functional groups, a poly glycol having a melting point of at least 55° C, and one or more second excipients.

The coating composition comprises an aqueous dispersion of a neutral ester copolymer without any functional groups. The aqueous dispersion of a neutral ester copolymer without any functional groups might be an ethyl acrylate and methyl methacrylate copolymer dispersion. Non-limiting examples of ethyl acrylate and methyl methacrylate copolymer dispersions include a 30% aqueous dispersion of a neutral copolymer based on ethyl acrylate and methyl methacrylate (e.g. Eudragit® NE30D), a 40% aqueous dispersion of a neutral copolymer based on ethyl acrylate and methyl methacrylate (e.g. Eudragit® NE40D), Eudragit® NM30D, Kollicoat® EMM30D, and combinations thereof. In at least one embodiment the neutral ester copolymer without any functional groups used in the controlled-release coating composition is Eudragit® NE30D, Eudragit® NE40D, or a mixture thereof. The neutral ester copolymer without any functional groups might be present in certain embodiments in an amount of from about 1 % to about 35% by weight of the coating composition, depending on the therapeutically active agent used and the controlled-release profile desired. In certain embodiments the neutral ester copolymer without any functional groups is present in an amount from about 20% to about 99.5% by dry weight of the coat. In other embodiments the neutral ester copolymer without any functional groups is present in an amount from about 25% to about 60% by dry weight of the coat. In still other embodiments the neutral ester copolymer without any functional groups is present in an amount from about 37% to about 50% by dry weight of the coat. In some embodiments, the neutral ester copolymer without any functional groups is present in an amount of about 38%, about 39%, about 40%, about 41%, about 42%, about 43%, about 44%, about 45%, about 46%, about 47%, about 48%, and about 49% by dry weight of the coat. In certain embodiments, the neutral ester copolymer without any functional groups is present in the coating composition in an amount of from about 0.4% to about 39.8% by dry weight of the tablet. in other embodiments in an amount of from about 0.8% to about 24% by dry weight of the tablet. In some other embodiments, the neutral ester copolymer without any functional groups is present in the coating composition in an amount of from about 2% to about 5.5% by dry weight of the tablet, for example, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4% by dry weight of the tablet.

In some embodiments, the controlled-release dosage form does not swell in a dimensionally unrestricted manner upon imbibition of water. In certain embodiments there is some swelling of the controlled-release dosage form in a dimensionally restricted manner upon imbibition of water. In certain embodiments the controlled-release coating restricts the swelling of the dosage form upon imbibition of water.

The coating composition also comprises a poly glycol with a melting point of at least about 55° C. The poly glycol with a melting point of at least about 55° C might be a polyethylene glycol with an average molecular weight ranging from about 4,000 Daltons to about 35,000 Daltons. Non-limiting examples of a poly glycol with a melting point of at least about 55° C that might be used with the coating compositions include polyethylene glycol 4000, polyethylene glycol 4600, polyethylene glycol 6000, polyethylene glycol 8000, polyethylene glycol 10000, polyethylene glycol 12000, polyethylene glycol 20000, polyethylene glycol 35000, and mixtures thereof. In certain embodiments, the poly glycol is selected from the group consisting of polyethylene glycol 6000, polyethylene glycol 8000, polyethylene glycol 10000, polyethylene glycol 12000, and mixtures thereof. In at least one embodiment the poly glycol used in the coating composition is polyethylene glycol 8000. The poly glycol might be present in certain embodiments in an amount of from about 0.1% to about 10% by weight of the coating composition. In certain embodiments the poly glycol is present in an amount of from about 0.5% to about 28% by dry weight of the coat. In other embodiments the poly glycol is present in an amount from about 4% to about 17% by dry weight of the coat. In still other embodiments the poly glycol is present in an amount from about 7.2% to about 15.2% by dry weight of the coat, for example, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 10.1%, about 10.2%, about 10.3%, about 10.4%, about 10.5%, about 10.6%, about 10.7%, about 10.8%, about 10.9%, about 11%, about 11.1%, about 11.2%, about 11.3%, about 11.4%, about 11.5%, about 11.6%, about 11.7%, about 11.8%, about 11.9%, about 12%, about 12.1%, about 12.2%, about 12.3%, about 12.4%, about 12.5%, about 12.6%, about 12.7%, about 12.8%, about 12.9%, about 13%, about 13.1%, about 13.2%, about 13.3%, about 13.4%, about 13.5%, about 13.6%, about 13.7%, about 13.8%, about 13.9%, about 14%, about 14.1%, about 14.2%, about 14.3%, about 14.4%, about 14.5%, about 14.6%, about 14.7%, about 14.8%, about 14.9%, about 15%, and about 15.1% by dry weight of the coat. In certain embodiments the poly glycol is present in the coating composition in an amount of from about 0.1% to about 11.2% by dry weight of the tablet. In other embodiments the poly

glycol is present in the coating composition in an amount of from about 0.1% to about 8% by dry weight of the tablet. In still other embodiments the poly glycol is present in the coating composition in an amount of from about 0.2% to about 2.8% by dry weight of the tablet, for example, about 0.3%, about 0.4%, about 0.5%, about 0.6%, about 0.7%, about 0.8%, about0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, and about 2.7% by dry weight of the tablet. Other suitable polyglycol derivatives having a melting point of at least about 55° C might be, but are not limited to, Poloxamer 188, Poloxamer 338, Poloxamer 407, Polyethylene Oxides, Polyoxyethylene Alkyl Ethers, Polyoxyethylene Stearates, and mixtures thereof.

In addition to the copolymers and the poly glycol, the coating composition comprises one or more other excipients. The excipients can include, but not limited to, anti-tacking agents, emulsifying agents, antifoaming agents, hydrophilic agents, flavorings, colorants, sweeteners etc, and any combination thereof. In some embodiments, excipients might affect the properties of the coat in a series of ways, and many substances used in coat formulations might thus be described as multifunctional. A skilled worker will know, based on his technical knowledge, which excipients are suitable for the desired controlled-release coating composition.

Hydrophilic agents might be included in the coat to promote wetting of the coat when in contact with gastrointestinal fluids. Such hydrophilic agents include hydrophilic water soluble polymers such as hydroxypropyl methylcellulose (HPMC) (e.g. Pharmacoat® 606 or Hypromellose), hydroxypropyl cellulose (HPC), methyl cellulose, hydroxyethyl cellulose, hydroxyethyl methylcellulose, polyvinylpyrrolidone (Povidone® or Kollidon®), polyvinyl alcohol, polyethylene oxide, vinylpyrrolidone-vinyl acetate copolymer (Kollidon® VA64), polyethylene glycol-polyvinyl alcohol copolymer (Kollicoat® IR), copolymers thereof, and combinations thereof. In at least one embodiment, HPMC is the hydrophilic agent used in the coating composition. In certain embodiments, the hydrophilic agent comprises a pH-dependent polymer, non-limiting examples of which include: Cellulose Acetate Phthalate (e.g. Aquacoat® CPD); Cellulose Acetate Trimellitate, Poly(methacrylic acid, ethyl acrylate) 1:1 (e.g. Eudragit® L30D-55); Kollicoat® MAE 30 D; Poly(methacrylic acid, ethyl acrylate) 1:1 (e.g. Eudragit® L100-55); Kollicoat® MAE 30 DP; Eudragit® FS 30D; Hypromellose Acetate Succinate LF, MF, HF grades (e.g. AQOAT®), Polyvinyl Acetate Phthalate, and mixtures thereof. If hydrophilic agents are to be included in the coat composition the agents might be present in certain embodiments in an amount from about 0.1% to about 10% by weight of the coating composition. In other embodiments from about 0.1% to about 5% by weight of the coating composition, and in still other embodiments from about 0.1% to about 3% by weight of the coating composition. In certain embodiments the hydrophilic agent is present in an amount of from greater than about 0% to about 35% by dry weight of the coat. In other embodiments the hydrophilic agent is present in an amount from about 8% to about 30% by dry weight of the coat. In still other embodiments the hydrophilic agent is present in an amount from about 12% to about 26% by dry weight of the coat, for example, about 13%, about 14%, about 15%, about 16%, about 17%, about 18%, about 19%, about20%, about21%, about22%, about23%, about 24%, and about 25% by dry weight of the coat. In certain embodiments the hydrophilic agent is present in the coating formulation in an amount of from about 0% to about 14% by dry weight of the tablet; in other embodiments in an amount of from about 0.2% to about 6% by dry weight of the tablet,; and in still other embodiments in an amount of from about 0.8% to about 2.5% by dry weight of the tablet; for example, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, and about 2.4% by dry weight of the tablet.

The tackiness of polymeric films is important for the coating of dosage forms and for the subsequent curing step (post coating thermal treatment). During coating with either cellulosic or acrylic polymers, an unwanted, and sometimes irreversible agglomeration of several granules or beads or, in the worst case, of the complete batch, might occur, especially at higher product processing temperatures. Accordingly, the addition of anti-tacking agents to coating formulations is desirable. The anti-tacking agents which can be used include but are not limited to adipic acid, magnesium stearate, calcium stearate, zinc stearate, hydrogenated vegetable oils, sterotex, glyceryl monostearate, talc (e.g. Talc 400), sodium benzoate, sodium lauryl sulfate, magnesium lauryl sulfate, and mixtures thereof. In at least one embodiment talc (e.g. talc 400) is used as the anti-tacking agent. Talc can also function as a wetting agent. Mixtures of the anti-tacking agents are operable. The amount of anti-tacking agent in the coating composition of certain embodiments can be in the range of from about 1% to about 15% by weight of the coating dispersion, and in certain embodiments from about 1% to about 7% by weight of the coating dispersion. In certain embodiments the anti-tacking agent is present in an amount of from greater than about 0% to about 50% by dry weight of the coat. In other embodiments the anti-tacking agent is present in an amount from about 2% to about 40% by dry weight of the coat. In still other embodiments the anti-tacking agent is present in an amount from about 10% to about 30% by dry weight of the coat; for example, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, and about 29% by dry weight of the coat. In certain embodiments the anti-tacking agent is present in the coating formulation in an amount of from about 0% to about 20% by dry weight of the tablet; in other embodiments in an amount of from about 0% to about 12% by dry weight of the tablet; and in still other embodiments in an amount of from about 0.6% to about 7% by dry weight of the tablet; for example, about 0.7%, about 0.8%, about 0.9%, about 1%, about 1.1%, about 1.2%, about 1.3%, about 1.4%, about 1.5%, about 1.6%, about 1.7%, about 1.8%, about

1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, and about 6.9% by dry weight of the tablet.

The anti-foaming agents, which might be included in the coating composition include silicon oil, simethicone (e.g. simethicone emulsion), and mixtures thereof. In at least one embodiment the anti-foaming agent is simethicone. The anti-foaming agent, if present, might be present in certain embodiments in an amount of up to about 0.5% by weight of the coat composition, and in certain other embodiments from about 0.1% to about 0.4% by weight of the coating composition. In certain embodiments the anti-foaming agent is present in an amount of from greater than about 0% to about 3% by dry weight of the coat. In other embodiments the anti-foaming agent is present in an amount from about 0.4% to about 2% by dry weight of the coat. In still other embodiments the anti-foaming agent is present in an amount from about 0.8% to about 1.5% by dry weight of the coat; for example, about 0.9%, about 1%, about 1.1%, about 1.2%, about 1.3%, and about 1.4% by dry weight of the coat. In certain embodiments the anti-foaming agent is present in the coating formulation in an amount of from about 0% to about 1.2% by dry weight of the tablet; in other embodiments in an amount of from about 0% to about 0.8% by dry weight of the tablet; and in still other embodiments in an amount of from about 0% to about 0.2% by dry weight of the tablet; for example, about 0.01%, about 0.02%, about 0.03%, about 0.04%, about 0.05%, about 0.06%, about 0.07%, about 0.08%, about 0.09%, about 0.10%, about 0.11%, about 0.12%, about 0.13%, about 0.14%, about 0.15%, about 0.16%, about 0.17%, about 0.18%, and about 0.19% by dry weight of the tablet.

The inclusion of an emulsifying agent (also called emulsifiers or emulgents) might be used to facilitate actual emulsification during manufacture of the coat, and also to provide emulsion stability during the shelf-life of the product. Emulsifying agents useful for the coat composition include, but are not limited to naturally occurring materials and their semi synthetic derivatives, such as the polysaccharides, as well as glycerol esters, cellulose ethers, sorbitan esters and polysorbates. Mixtures are operable. In at least one embodiment the emulsifying agent used is Polysorbate 80 (polyoxyethylene sorbitan mono-oleate), (e.g. Tween® 80). The emulsifying agent or agents, if present, might be present in certain embodiments in an amount of from greater than 0% to about 0.5% by weight of the coat composition. In at least one embodiment the emulsifying agent is present in an amount of from about 0.1% to about 0.3% by weight of the coat composition. In certain embodiments the emulsifying agent is present in an amount of from greater than about 0% to about 2% by dry weight of the coat. In other embodiments the emulsifying agent is present in an amount from about 0.1% to about 1% by dry weight of the coat. In still other embodiments the emulsifying agent is present in an amount from about 0.25% to about 0.75% by dry weight of the coat; for example, including about 0.30%, about 0.35%, about 0.40%, about 0.45%, about 0.50%, about 0.55%, about 0.60%, about 0.65%, and about 0.70% by dry weight of the coat. In certain embodiments the emulsifying agent is present in the coating formulation in an amount of from greater than about 0% to about 0.8% by dry weight of the tablet; in other embodiments in an amount of from greater than about 0% to about 0.4% by dry weight of the tablet; and in still other embodiments in an amount of from greater than about 0% to about 0.2% by dry weight of the tablet; for example, about 0.01%, about 0.02%, about 0.03%, about 0.04%, about 0.05%, about 0.06%, about 0.07%, about 0.08%, about 0.09%, about 0.10%, about 0.11%, about 0.12%, about 0.13%, about 0.14%, about 0.15%, about 0.16%, about 0.17%, about 0.18%, and about 0.19% by dry weight of the tablet.

Any permitted colorants in a film coat formula are invariably water-insoluble colors (pigments). Pigments have certain advantages over water-soluble colors in that they tend to be more chemically stable towards light, provide better opacity and covering power, and optimize the impermeability of a given film to water vapor. Examples of suitable colorants include, but are not limited to iron oxide pigments, titanium dioxide, and aluminum Lakes. Mixtures are operable. In at least one embodiment the pigment or colorant used is titanium dioxide. The pigment or colorant, if present, might be present in certain embodiments in an amount of from about 0.1% to about 10% by weight of the coat composition. In at least one embodiment the colorant is present in an amount of from about 0.1% to about 5% by weight of the coat composition. In at least one other embodiment the colorant is present in an amount of from about 0.1% to about 2% by weight of the coat composition. In certain embodiments the colorant is present in an amount of from greater than about 0% to about 20% by dry weight of the coat. In other embodiments the colorant is present in an amount from greater than about 0% to about 10% by dry weight of the coat. In still other embodiments the colorant is present in an amount from about 2.2% to about 6.2% by dry weight of the coat; for example, including about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, and about 6.1% by dry weight of the coat. In certain embodiments the colorant is present in the coating formulation in an amount of from greater than about 0% to about 8% by dry weight of the tablet; in other embodiments in an amount of from greater than about 0% to about 5% by dry weight of the tablet; and in still other embodiments in an amount of from greater than about 0% to about 1% by dry weight of the tablet; for example, including

about 0.1%, about 0.2%, about 0.3%, about 0.4%, about 0.5%, about 0.6%, about 0.7%, about 0.8%, and about 0.9% by dry weight of the tablet.

In at least one embodiment the second excipients in the controlled-release coating comprises at least one of a neutral ester copolymer without any functional groups (e.g. Eudragit® NE30D, Eudragit® NE40D, Eudragit® NM30D, Kollicoat® EMM30D, or a mixture thereof), HPMC (e.g. Pharmacoat®606), talc (e.g. Talc 400), polyethylene glycol (e.g. polyethylene glycol 4000, polyethylene glycol 4600, polyethylene glycol 6000, polyethylene glycol 8000, polyethylene glycol 10000, polyethylene glycol 12000, polyethylene glycol 20000, polyethylene glycol 35000, or a mixture thereof), simethicone, polysorbate 80, titanium dioxide, and mixtures thereof.

In at least one embodiment, the stable controlled-release coating hydrates when placed into water. In at least one embodiment the dosage form that is coated with the controlled- release coating floats in water. In at least one embodiment, the controlled-release dosage form, upon oral administration to a patient, provides controlled-release of an effective amount of the active drug to at least one region of the patient's upper gastrointestinal tract (e.g. the stomach).

In some embodiments, the controlled-release coating is formed by a process that does not involve the use of an organic solvent. In such embodiments the controlled-release coating composition is aqueous based and not solvent based (termed "AQ" in certain examples of dosage forms coated with the aqueous based controlled-release coating). In some embodiments, the controlled-release coating is formed by a process that are solvent based (e.g. "PharmaPASS™" composition).

The coating composition can be applied onto a core comprising an effective amount of the therapeutically active agent by a process, which involves the atomization (spraying) of the coating composition (solution or suspension) onto a bed of the tablet cores. Some examples of equipment suitable for film coating include: ACCELA COTA® (Manesty Machines, Liverpool, UK), HI-COATER® (Freund Company, Japan), DRIACOATER™ (Driam Metallprodukt GmbH, Germany), HTF/150™ (GS, Italy), and IDA™ (Dumoulin, France). Examples of units that function on a fluidized-bed principle include: AEROMATIC™ (Fielder, Switzerland and UK) and GLATT AG™ (Switzerland). In at least one embodiment the apparatus used is the ACCELA COTA®.

The coating composition is delivered to the coating apparatus from a peristaltic pump at the desired rate and sprayed onto the rotating or fluidizing tablet cores. The tablet cores are pre- warmed to about 30° C. During the coating process, the product temperature range is maintained between about 25° C and about 35° C by adjusting the flow rate of the inlet and outlet air, temperature of the inlet air and spray rate. A single layer of the coating composition is applied and once spraying is complete, the coated tablet cores are dried between about 30° C to about 40° C for about 3 to about 5 minutes at a low pan speed and low air flow. The pan is readjusted to jog speed, and drying continued for about 12 to about 15 minutes.

The coated tablet cores are placed onto a tray and cured (post coating thermal treatment) in an electrical or steam oven at a temperature above the temperature of the melting point of the polyethylene glycol or derivative thereof. In at least one embodiment the curing temperature is greater than the melting point of the polyethylene glycol or derivative thereof. In at least one embodiment the curing time is from about 2 to about 7 hours. The cured coated tablets are subsequently cooled to about room temperature.

In certain other embodiments, the coated tablet cores are placed onto a coating pan and cured at two-stages. During the first stage, the coated tablets are cured at a first curing temperature (for example, in certain embodiments from between about 50° C to about 59° C) for a period of time (for example, in certain embodiments from about 15 minutes to about 90 minutes; and in at least one embodiment for about 60 minutes). During the second stage, the coated tablets are cured at a second curing temperature that is at least equal to or greater than the melting point of the poly glycol (for example, in certain embodiments from between about 60° C to about 70° C) for an additional period of time (for example, in certain embodiments from about 30 minutes to about 180 minutes; and in at least one embodiment for about 120 minutes). In at least one embodiment the two- stage curing of the coated tablets reduces non-functional defects on the tablet caused by the curing process. In at least one embodiment the two-stage curing process substantially eliminates non- functional defects on the tablet caused by the curing process. Non-functional defects on the dosage form caused by the curing process can include visual defects in the coating (e.g. poor color uniformity, and/or dull appearance), defects in the surface of the coating (e.g. roughness in the surface of the coating, and/or wrinkles in the coating), and sticking of the tablets to each other and/or to the coating pan. In addition, the reduced defects in color and smoothness of the tablets allows for improved printing of the tablets

In some embodiments, the coating formulation is used to coat a variety of the calcium alpha-ketoglutarate cores and might be adjusted to obtain a desired drug release profile. The length and time for the delay is controlled by rate of hydration and the thickness of the coat. The drug release rate subsequent to the delay is determined by the thickness and permeability of the hydrated coat. Thus, it is possible to regulate the rate of hydration and permeability of the coat so that the desired controlled-release drug profile might be achieved. There is no preferred coat thickness, as this will depend on the drug being used in the core and also the controlled-release profile desired.

Other parameters in combination with the thickness of the coat include varying the concentrations of some of the ingredients of the stable coat composition and/or varying the curing temperature and length of curing the coated tablet

cores. The skilled artisan will know which parameters or combination of parameters to change for a desired controlled-release profile.

Immediate-Release Coating Formulations

In some embodiments, the immediate-release coating comprises the calcium alpha-ketoglutarate and an additional agent described herein. In some embodiments, an effective amount of the immediate-release active agent in immediate-release form is coated onto the formulations described herein. For example, where the extended release of the calcium alpha-ketoglutarate from the formulation is due to a controlled-release coating, the immediate-release layer of the additional agent would be overcoated on top of the controlled-release coating. In some embodiments, the immediate-release layer of the additional agent is coated onto the surface of substrates wherein the calcium alpha-ketoglutarate is incorporated in a controlled-release matrix. Where a plurality of the sustained release substrates comprising an effective unit dose of the calcium alpha-ketoglutarate (e.g., multiparticulate systems including pellets, spheres, beads and the like) are incorporated into a hard gelatin capsule, the side-effect-reducing compound might be incorporated into the gelatin capsule via inclusion of the sufficient amount of immediate-release antihistamine or antiemetic as a powder or granulate within the capsule. Alternatively, the gelatin capsule itself might be coated with an immediate-release layer of the additional agent.

A coating containing the immediate-release of the additional agent such as antihistamine or antiemetic might be added to the outside of the controlled-release tablet cores to produce a final dosage form. Such a coating might be prepared by mixing compounds like promethazine with polyvinylpyrrolidone (PVP) 29/32 or hydroxypropyl methylcellulose (HPMC) and water/isopropyl alcohol and triethyl acetate. Such an immediate-release coating might be spray coated onto the tablet cores. The immediate-release coating might also be applied using a press- coating process with a blend consisting of 80% by weight promethazine and 20% by weight of lactose and hydroxypropyl methylcellulose type 2910.

In some embodiments, the immediate-release/controlled-release dosage forms described herein form bi-layered tablets, which comprise a first layer and a second layer. In some embodiments of the bi-layered tablet, the first layer is an immediate-release layer and/or the second layer is a controlled-release layer.

Formulations and Routes of Administration

Sustained-release compositions of the calcium alpha-ketoglutarate disclosed herein might be formulated in a conventional manner using one or more physiologically acceptable carriers including excipients and auxiliaries which facilitate processing of the active compounds into preparations which might be used pharmaceutically. Proper formulation is dependent upon the route of administration chosen. Additional details about suitable excipients for compositions described herein might be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

The compositions of the calcium alpha-ketoglutarate described herein are administered to a subject by multiple administration routes, including but not limited to, oral, parenteral (e.g., intravenous, subcutaneous, intramuscular), intranasal, inhalation, buccal, topical, rectal, or transdermal administration routes. In some embodiments, compositions described herein, which include the calcium alpha-ketoglutarate, are formulated into any suitable dosage form, including but not limited to, emulsions suitable for injection, nanosuspensions suitable for injection, aqueous oral dispersions, liquids, gels, syrups, elixirs, slurries, suspensions, aerosols, controlled- release formulations, fast melt formulations, effervescent formulations, lyophilized formulations, tablets, powders, pills, dragees, capsules, delayed release formulations, extended release formulations, pulsatile release formulations, multiparticulates formulations, and mixed immediate-release and controlled-release formulations.

In some embodiments, the calcium alpha-ketoglutarate is calcium alpha-ketoglutarate monohydrate. In some embodiments, the composition is an oral formulation, a buccal formulation, a nasal formulation, or an inhalation formulation. In some embodiments, the composition is in a form of a tablet or capsule.

In some embodiments, the composition for oral use is a tablet, (including a suspension tablet, a fast-melt tablet, a bite-disintegration tablet, a rapid-disintegration tablet, an effervescent tablet, or a caplet), a pill, a powder (including a sterile packaged powder, a dispensable powder, or an effervescent powder), a capsule (including both soft or hard capsules, e.g., capsules made from animal-derived gelatin or plant-derived HPMC, or "sprinkle capsules"), solid dispersion, solid solution, bioerodible dosage form, controlled-release formulations, pulsatile release dosage forms, multiparticulate dosage forms, pellets, granules, or an aerosol. In some embodiments, the composition for oral use is a solid dosage form, e.g., tablets, effervescent tablets, and capsules. In some embodiments, the solid dosage form are prepared by mixing particles of the calcium alpha- ketoglutarate, with one or more pharmaceutically acceptable excipients

to form a bulk blend composition. When referring to these bulk blend compositions as homogeneous, it is meant that the particles of the calcium alpha-ketoglutarate, are dispersed evenly throughout the composition so that the composition might be subdivided into equally effective unit dosage forms, such as tablets, pills, and capsules. The individual unit dosages might also include film coatings, which disintegrate upon oral ingestion or upon contact with diluent.

For oral administration, the compositions disclosed herein might be formulated readily by combining the active compound(s) with pharmaceutically acceptable carriers well known in the art. Such carriers enable the compositions disclosed herein to be formulated as tablets, including chewable tablets, pills, dragees, capsules, lozenges, hard candy, liquids, gels, syrups, slurries, powders, suspensions, elixirs, wafers, and the like, for oral ingestion by a patient to be treated. Such formulations might comprise pharmaceutically acceptable carriers including solid diluents or fillers, sterile aqueous media and various non-toxic organic solvents. Generally, the compositions disclosed herein will be included at concentration levels ranging from about 0.5%, about 5%, about 10%, about 20%, or about 30% to about 50%, about 60%, about 70%, about 80% or about 90% by weight of the total composition of oral dosage forms, in an amount sufficient to provide a desired unit of dosage.

Additional Agents

In some embodiments, the sustained-release composition of the calcium alpha-ketoglutarate further comprises one or more agents. In some embodiments, one or more agents are selected from the group consisting of surfactants, preservatives, flavoring agents, vitamins, antioxidants, and sweetening agents.

In some embodiments, the composition comprises a vitamin. In some embodiments, the vitamin is vitamin A, vitamin B, vitamin C, or vitamin D.

In some embodiments, the vitamin is vitamin A In some embodiments, the amount of vitamin A is from 500 IU to 5000 IU. In some embodiments, the amount of vitamin A is from 1000 IU to 3000 IU. In some embodiments, the amount of vitamin A is from 1000 IU to 2000 IU. In some embodiments, the amount of vitamin A is at least 500 IU. In some embodiments, the amount of vitamin A is at most 5,000 IU. In some embodiments, the amount of vitamin A is from 500 IU to 750 IU, 500 IU to 1,000 IU, 500 IU to 1,500 IU, 500 IU to 2,000 IU, 500 IU to 2,500 IU, 500 IU to 3,000 IU, 500 IU to 3,500 IU, 500 IU to 4,000 IU, 500 IU to 4,500 IU, 500 IU to 5,000 IU, 750 IU to 1,000 IU, 750 IU to 1,500 IU, 750 IU to 2,000 IU, 750 IU to 2,500 IU, 750 IU to 3,000 IU, 750 IU to 3,500 IU, 750 IU to 4,000 IU, 750 IU to 4,500 IU, 750 IU to 5,000 IU, 1,000 IU to 1,500 IU, 1,000 IU to 2,000 IU, 1,000 IU to 2,500 IU, 1,000 IU to 3,000 IU, 1,000 IU to 3,500 IU, 1,000 IU to 4,000 IU, 1,000 IU to 4,500 IU, 1,000 IU to 5,000 IU, 1,500 IU to 2,000 IU, 1,500 IU to 2,500 IU, 1,500 IU to 3,000 IU, 1,500 IU to 3,500 IU, 1,500 IU to 4,000 IU, 1,500 IU to 4,500 IU, 1,500 IU to 5,000 IU, 2,000 IU to 2,500 IU, 2,000 IU to 3,000 IU, 2,000 IU to 3,500 IU, 2,000 IU to 4,000 IU, 2,000 IU to 4,500 IU, 2,000 IU to 5,000 IU, 2,500 IU to 3,000 IU, 2,500 IU to 3,500 IU, 2,500 IU to 4,000 IU, 2,500 IU to 4,500 IU, 2,500 IU to 5,000 IU, 3,000 IU to 3,500 IU, 3,000 IU to 4,000 IU, 3,000 IU to 4,500 IU, 3,000 IU to 5,000 IU, 3,500 IU to 4,000 IU, 3,500 IU to 4,500 IU, 3,500 IU to 5,000 IU, 4,000 IU to 4,500 IU, 4,000 IU to 5,000 IU, or 4,500 IU to 5,000 IU. In some embodiments, the amount of vitamin A is about 500 IU. In some embodiments, the amount of vitamin A is about 750 IU. In some embodiments, the amount of vitamin A is about 1,000 IU. In some embodiments, the amount of vitamin A is about 1,500 IU. In some embodiments, the amount of vitamin A is about 2,000 IU. In some embodiments, the amount of vitamin A is about 2,500 IU. In some embodiments, the amount of vitamin A is about 3,000 IU. In some embodiments, the amount of vitamin A is about 3,500 IU. In some embodiments, the amount of vitamin A is about 4,000 IU. In some embodiments, the amount of vitamin A is about 4,500 IU. In some embodiments, the amount of vitamin A is about 5,000 IU.

In some embodiments, the vitamin is vitamin D. In some embodiments, the amount of vitamin D is from 100 IU to 3000 IU. In some embodiments, the amount of vitamin D is from 200 IU to 2000 IU. In some embodiments, the amount of vitamin D is from 250 IU to 1000 IU. In some embodiments, the amount of vitamin D is from 100 IU to 3,000 IU. In some embodiments, the amount of vitamin D is at least 100 IU. In some embodiments, the amount of vitamin D is at most 3,000 IU. In some embodiments, the amount of vitamin D is from 100 IU to 250 IU, 100 IU to 500 IU, 100 IU to 750 IU, 100 IU to 1,000 IU, 100 IU to 1,250 IU, 100 IU to 1,500 IU, 100 IU to 1,750 IU, 100 IU to 2,000 IU, 100 IU to 2,500 IU, 100 IU to 3,000 IU, 250 IU to 500 IU, 250 IU to 750 IU, 250 IU to 1,000 IU, 250 IU to 1,250 IU, 250 IU to 1,500 IU, 250 IU to 1,750 IU, 250 IU to 2,000 IU, 250 IU to 2,500 IU, 250 IU to 3,000 IU, 500 IU to 750 IU, 500 IU to 1,000 IU, 500 IU to 1,250 IU, 500 IU to 1,500 IU, 500 IU to 1,750 IU, 500 IU to 2,000 IU, 500 IU to 2,500 IU, 500 IU to 3,000 IU, 750 IU to 1,000 IU, 750 IU to 1,250 IU, 750 IU to 1,500 IU, 750 IU to 1,750 IU, 750 IU to 2,000 IU, 750 IU to 2,500 IU, 750 IU to 3,000 IU, 1,000 IU to 1,250 IU, 1,000 IU to 1,500 IU, 1,000 IU to 1,750 IU, 1,000 IU to 2,000 IU, 1,000 IU to 2,500 IU, 1,000 IU to 3,000 IU, 1,250 IU to 1,500 IU, 1,250 IU to 1,750 IU, 1,250 IU to 2,000 IU, 1,250 IU to 2,500 IU, 1,250 IU to 3,000 IU, 1,500 IU to 1,750 IU, 1,500 IU to 2,000 IU, 1,500 IU to 2,500 IU, 1,500 IU to 3,000 IU, 1,750 IU to 2,000 IU, 1,750 IU to 2,500 IU, 1,750 IU to 3,000 IU, 2,000 IU to 2,500 IU, 2,000 IU to 3,000 IU, or 2,500 IU to 3,000 IU. In some embodiments, the amount of vitamin D is about 100 IU. In some embodiments, the amount of vitamin D is about 250 IU. In some embodiments, the amount of vitamin D is about 500 IU. In some embodiments, the amount of vitamin D is about 750 IU. In some embodiments, the amount of vitamin D is about 1,000 IU. In some embodiments, the amount of vitamin D is about 1,250 IU. In some embodiments, the amount of vitamin D

is about 1,500 IU. In some embodiments, the amount of vitamin D is about 1,750 IU. In some embodiments, the amount of vitamin D is about 2,000 IU. In some embodiments, the amount of vitamin D is about 2,500 IU. In some embodiments, the amount of vitamin D is about 3,000 IU.

In some embodiments, the vitamin is nicotinamide riboside (NR). In some embodiments, the vitamin is nicotinamide mononucleotide (NMN).

In some embodiments, the composition comprises an antioxidant. In some embodiments, the antioxidant is fisetin.

EXAMPLES

The following examples are provided for illustrative purposes only, and are intended to be purely exemplary of the disclosure and are not intended to limit the scope of the claims provided herein.

EXAMPLE 1. General Manufacturing Procedure for Calcium alpha-Ketoglutarate Sustained Release Tablets. Illustrative tablets include 350-450 mg of alpha-ketoglutaric acid by weight based on the free acid. Further, illustrative tablets include 450-650 mg of alpha-ketoglutarate by weight based on the salt form, and hydrate form. Further, illustrative tablets include 150-250 mg by weight of calcium when the alpha-ketoglutarate is a calcium salt. Uncoated tablets have an average weight of about 1000-1000 mg, including about 1030 mg. Coated tablets have an average weight of about 1020-1120 mg, including about 1060 mg. Illustrative tablet cores include the following:

Tablet Core 1

| Item | Ingredients | Qty/Cap (mg) | Qty/Batch (gm) |
|---|---|---|---|
| Stage: Dry Mix | | | |
| 1 | Calcium alpha-ketoglutarate | 500 | 2500 |
| 2 | Microcrystalline cellulose PH 102 Avicel | 190.8 | 954 |
| 3 | Lactose monohydrate DCL-11 | 137.2 | 686 |
| 4 | HPMC K4M Methocel | 58 | 290 |
| 5 | HPMC K15M Methocel | 87 | 435 |
| 6 | Colloidal silicon dioxide | 10 | 50 |
| 7 | Magnesium stearate | 10 | 50 |
| Stage: Lubrication | | | |
| 9 | Colloidal silicon dioxide | 19.75 | 98.75 |
| 10 | Magnesium stearate | 17.25 | 86.25 |
| Average weight | | 1030 | 5150 |

Tablet Core 2

| Item | Ingredients | Qty/Cap (mg) | Qty/Batch (gm) |
|---|---|---|---|
| Stage: Dry Mix | | | |
| 1 | Calcium alpha-ketoglutarate | 525 | 2625 |
| 2 | Microcrystalline cellulose PH 102 Avicel | 190.8 | 954 |
| 3 | Lactose monohydrate DCL-11 | 137.2 | 686 |
| 4 | HPMC K4M Methocel | 58 | 290 |
| 5 | HPMC K15M Methocel | 87 | 435 |
| 6 | Colloidal silicon dioxide | 10 | 50 |
| 7 | Magnesium stearate | 10 | 50 |
| Stage: Lubrication | | | |
| 9 | Colloidal silicon dioxide | 19.75 | 98.75 |
| 10 | Magnesium stearate | 17.25 | 86.25 |

(continued)

| Stage: Lubrication | | |
|---|---|---|
| Average weight | 1030 | 5150 |

Illustrative coatings include the following:

Coating

| Item | Ingredients | Qty/Cap (mg) | Qty/Batch (gm) |
|---|---|---|---|
| 1 | Instamoistshield white (ICMS 2398) | 31 | 155 |
| 2 | Isopropyl alcohol | qs | 1200 mL |
| 3 | Methylene chloride | qs | 1750 mL |

CALCULATION

For example, calcium alpha-ketoglutarate monohydrate corresponding to:

alpha-ketoglutaric acid= 350 mg;
Conversion factor = 1.26 (for alpha-ketoglutarate anhydrous);
Qty. after conversion= 441 mg;
Std LOD = 10%; Std Assay = 98%;
Std. Calculation: (441 x 100 x 100)/(98 x (100-10)) = 500 mg;
Also contains elemental calcium= 19.5 to 22%;
Considering lowest value= 19.5%;
500 mg CAKG contains 97.5 mg elemental calcium.

Standard Manufacturing Procedure

1. WEIGHING

Weigh all ingredients as per quantity given in manufacturing formula.

2. SIFTING

Mix HPMC K4 290.0 gm and HPMC KI5 435.0 gm in double cone blender for 5 min and sift through 40# sieve. Sift lactose monohydrate 686.0 gm and microcrystalline cellulose 954.0 gm through 40# sieve. Then mix with calcium alpha-ketoglutarate monohydrate 2500.0 gm for 2 min and sift through 30# sieve. Sift colloidal silicon dioxide 50.0 gm through 40# sieve and magnesium stearate 50.0 gm by 60# sieve. Collect each sifted material individually in polybag. (The temperature and RH should be NMT 27°C and 40% RH through the process)

3. MIXING & GRANULATION

Mix above sifted ingredients (HPMC K4 and HPMC KI 5, calcium alpha-ketoglutarate monohydrate, lactose monohydrate, colloidal silicon dioxide and microcrystalline cellulose) in double cone blender (capacity 18 Liters) for 25 min at 16 rpm. Add magnesium stearate to above blend and mix for 5 min in double cone blender (capacity 18 Liters).

4. SLUGGING

Slug dry mix material obtained from previous step into tablet using suitable punch size with compression machine to achieve tablets. Punch description: 19 6 x 8.4 mm, capsule shaped, standard concave, plain punch. Hardness: 2-5 $kg/cm^2$.

5. DESLUGGING

Mill the tablets by using 6.0 mm S.S screen and final sizing of granules through 18# sieve. Note: (Slug and deslug to be done twice to obtain granules with suitable flow properties).

Deslugged granules parameters (Target):

1) Bulk Density - 0.6 to 0.7 gm/ml;
2) Tapped Density - 0 9 to 1.0 gm/ml;
3) Carr's Index - 25 - 30%; and
4) Hausner Ratio - 1.3 to 1.4.

6. LUBRICATION

Check intactness of sieve before use. Sift colloidal silicon dioxide 98.75 gm through 40# sieve. Sift magnesium stearate 86.25 gm through 60 #sieve.Mix de-slugged mass of step 5 with colloidal silicon dioxide in double cone blender for 5 mins. Add magnesium stearate to above blend for 5 mins in double cone blender. Collect lubricated granules in double polyethylene bags and keep tightly closed. In some embodiments, one or more of flavoring agents, vitamins, antioxidants, and sweetening agents are added to the composition during the lubrication step along with magnesium stearate and/or colloidal silicon dioxide.

7. COMPRESSION OF TABLETS

AMBIENT PARAMETERS:

1) RH-NMT 40%;
2) Temperature - NMT 27°C;

Air Pressure Differential - NLT 10 Pascal;
*Type of punch: 'D' Tooling;
19.6 x 8.4 mm, capsule shaped, standard concave. Plain punches.

Check complete rotation of the turret by turning hand wheel followed by electric operation. Feed granules and set machine as per desired specifications. Check tablet from one complete rotation.

8. FILM COATING OF TABLET

Add to disperse Instamoistshield white (ICMS 2398) into given quantity of isopropyl alcohol under stirring. Stir further for 5 min. Add methylene chloride into given quantity of above step, under continuous stirring, further stir for 40-45 min to get white colored viscous lump free suspension. Above suspension pass through colloidal mill by "O" adjustment gap for 15 min. Filter coating suspension through 200 mesh nylon cloths. Switch on exhaust and apply film coating suspension to the tablets using a clean spray gun assembly (ensure elegance) continue stirring of coating suspension during coaling of tablet. Dry film coated tablets sufficiently after proper weight build up (3 00 %). Target average weight of film coated tablets= 1060.0 mg. Once target weight gain achieved, dry film coated tablets in coating pan for 30 min.

EXAMPLE 2. Dissolution Profile of the Calcium alpha-Ketoglutarate Sustained-Release

[Dissolution Parameters]

Media: 900 mL simulated gastric fluid (SGF) without enzyme
Apparatus: Paddle (with sinker)
Speed: 75 rpm
Time: 1$^{st}$ hour, 4$^{th}$ hour, 8$^{th}$ hour, and 12$^{th}$ hours.

[Chromatographic conditions]

Column: Inertsil ODS, 3V (250 x 4.6 mm) 5 um or equivalent;
Flow rate: 0.8 mL/min
Wavelength: 210 nm
Injection volume: 10 uL
Oven Temperature: 30°C
Sample cooler Temperature: 15°C
Elution Mode: Isocratic

Simulated gastric fluid (SGF) without enzyme. (Dissolution Medium): weigh and transfer 2.0 gm of sodium chloride and 7.0 mL of concentrated hydrochloric acid into 1000 mL water and mix.
Buffer solution: dissolve 2.72 gm potassium dihydrogen orthophosphate in 1000 mL of water and add 2.0 mL of orthophosphoric acid.
Mobile phase: prepare a mixture 95 volume buffer and 5 volume of acetonitrile mix filter and degas before use.
Standard solution: weigh and transfer accurately 55 mg calcium alpha-ketoglutarate working standard into a 100 mL dry volumetric flask add 50 mL of dissolution medium. Dissolve and makeup the volume with dissolution medium and mix.
Test solution: set dissolution parameters and place one tablet in to each vessel taking care to exclude air bubbles from the surface of the tablets and immediately start the apparatus. After $1^{st}$ hour, $4^{th}$ hour, $8^{th}$ hour, and $12^{th}$ hours withdraw IO mL of the sample of medium and replace with drawn volume by fresh fluid. Filter withdraw sample through 0.45-micron nylon syringe filter; discard first few mL of the filtrate.
System suitability: inject five replicate injection of standard solution. The relative standard deviation for replicate injection is not more than 2 %.
Procedure: inject 10 uL of each test solution to liquid chromatography and record the chromatograms. Measure the responses for calcium alpha-ketoglutarate peaks. Calculate the percentage drug release of calcium alpha-ketoglutarate from the calcium alpha-ketoglutarate peak areas of standard and test percentage potency of working standard used.

Sequence of injection:

| Sequence Table | | |
|---|---|---|
| # | Sample | No. of injection |
| 1 | Blank | 1 |
| 2 | Reference Solution | 5 |
| 3 | Test Solution | 6 |
| 4 | Bracketing Standard | I |

Calculation:

$$(AT/AS) \times (W/100) \times (900/LC) \times (p/100) \times 100$$

wherein,

AT - Peak area of calcium alpha-ketoglutarate in the chromatogram obtained from each injection of test solution;
AS - Average peak area of calcium alpha-ketoglutarate in the chromatogram obtained from five replicate injections of standard solution;
WS - Weight of calcium alpha-ketoglutarate working standard in mg; LC - Label claim;
P - Potency of calcium alpha-ketoglutarate working standard on as such basis.

Correction factor:

(Withdrawn volume x % dissolved(% label claim) at preceding time interval)/Volume of dissolution medium
For 1st hour time point=% dissolved after 1 hour;
For $4^{th}$ hour time point= Correction factor for 1st hour+% dissolved after $4^{th}$ hour;
For $8^{th}$ hour time point= Correction factor for 1st hour+ correction factor for $4^{th}$ hour+% dissolved after $8^{th}$ hour;
For $12^{th}$ hour time point= Correction factor for 1st hour+ correction factor for $4^{th}$ hour+ correction factor for $8^{th}$ hour+% dissolved after $12^{th}$ hour;

Results:

Lot A (Dissolution,%)

| Time, h | Sample I | Sample 2 | Sample 3 | Sample 4 | Sample 5 | Sample 6 | Average |
|---|---|---|---|---|---|---|---|
| 1 | 18.22 | 15.58 | 16.3 | 14.89 | 16.44 | 15.58 | 16.17 |
| 4 | 45.97 | 48.99 | 43.58 | 44.29 | 43.3 | 44.94 | 45.18 |
| 8 | 72.12 | 68.41 | 67.41 | 70.55 | 71.9 | 69.99 | 70.06 |
| 12 | 90.5 | 89.51 | 89.57 | 91.66 | 90.85 | 86.34 | 89.74 |

Lot B (Dissolution,%)

| Time, h | Sample 1 | Sample 2 | Sample 3 | Sample 4 | Sample 5 | Sample 6 | Average |
|---|---|---|---|---|---|---|---|
| 1 | 18.19 | 16.06 | 15.89 | 15.39 | 14.94 | 14.23 | 15.78 |
| 4 | 43.61 | 47.6 | 42.01 | 43.29 | 44.98 | 44.01 | 44.25 |
| 8 | 70.45 | 69.68 | 69.97 | 70.82 | 74.84 | 69.32 | 70.85 |
| 12 | 91.3 | 85.11 | 87.37 | 87.05 | 87.2 | 87.44 | 87.58 |

METHOD EXAMPLE 1. Increasing healthspan and/or lifespan in mice. 18-month old non-genetically altered mice are fed a control diet, optionally a control diet plus an immediate release formulation (positive control), or a control diet plus a composition described herein (treatment group). The mice are not sacrificed but allowed to die naturally. The lifespan of the mice is compared between the treatment groups and control groups, and optionally the positive control groups. In addition to lifespan determination, the frailty of the mice is determined throughout the study by a non-invasive frailty index, composed of 31 observations, adapted from a clinically relevant frailty index. Compositions described herein increase lifespan, healthspan, and or improve frailty compared to control and positive control groups in both genders. Additional details for the Example are described in Lucanic, M., Lithgow, G. J., & Alavez, S. (2013). Pharmacological lifespan extension of invertebrates. Ageing research reviews, 12(1), 445-458; Searle, S. D., Mitnitski, A., Gahbauer, E. A., Gill, T. M., & Rockwood, K. (2008). A standard procedure for creating a frailty index. BMC geriatrics, 8(1), 24; and Whitehead (2014.).

METHOD EXAMPLE 2. Increasing healthspan and/or lifespan in human (male and female). Compositions described here are administered either orally or parenterally to male and female volunteers, and compared to a control group administered placebo, and optionally compared to a positive control group administered commercially available Ca-AKG, such as immediate release Ca-AKG. Volunteers are monitored for lifespan, healthspan, and frailty using one or more of the conventional one or more of the 31 frailty index observations. The study is an open label study compare the treatment group and control group, and optionally the positive control group. Volunteers are assessed at three timepoints: Study Initiation (Day 1), Month 3, and Month 6. At each visit blood pressure, heart rate, weight, safety labs, CRP, blood chemistries, Hemoglobin A1C, and uric acid levels are obtained, and a study questionnaire is completed by each participant.

Blood chemistry is used to calculate the biological age of the participant by one or more published algorithms, such as the method described in Belsky, D. W., Caspi, A., Houts, R., Cohen, H. J., Corcoran, D. L., Danese, A., & Sugden, K. (2015). Quantification of biological aging in young adults. Proceedings of the National Academy of Sciences, 112(30), E4104-E4110.

In one illustrative study design, volunteers are assigned to a control, optional positive control, or treatment group, and each volunteer takes two tablets per day for six months. The control group takes placebo, the positive control group takes an immediate release formulation, and the treatment group takes two tablets as follows:

Males: tablets comprising 500 mg of calcium alpha-ketoglutarate monohydrate; 450 mcg of retinyl palmitate; and one or more release-modifying agents;
Females: tablets comprising 500 mg of calcium alpha-ketoglutarate monohydrate;
12.5 mcg (500 IU) of cholecalciferol; and one or more release-modifying agents. One or more primary outcomes are evaluated for each group. The primary outcome is determined by measuring the following vital signs and blood chemistries:

Vital signs

Height
Weight
Blood Pressure
Heart rate

Complete Blood Count

WBC (White Blood Cell Count)
Hemoglobin
Hematocrit
Platelet Count /L
MCV (Mean Corpuscular Volume)
MCH (Mean Corpuscular Hemoglobin)
MCHC (Mean Corpuscular Hemoglobin Concentration)
RBC (Red Blood Cell Count)
RDW-CV (Red Cell Distribution Width)
MPV (Mean Platelet Volume)
Neutrophils
Neutrophil %
Lymphocyte Count
Lymphocyte %
Monocyte Count
Monocyte %
Eosinophils
Eosinophil %
Basophils
Basophil%

Comprehensive Metabolic Panel

Sodium
Potassium
Chloride
Carbon Dioxide ($CO_2$)
Urea Nitrogen (BUN)
Creatinine
Glucose
Calcium
AST (Aspartate Aminotransferase)
ALT (Alanine Aminotransferase)

ALP (Alkaline Phosphatase)
Bilirubin, Total
Alkaline Phosphatase
Albumin
Protein, Total
Anion Gap
Glomerular Filtration Rate (eGFR),
MDRD Estimate

Lipid Panel

Total Cholesterol
LDL (Low Density Lipoprotein) Cholesterol
HDL (High Density Lipoprotein) Cholesterol
Triglyceride

Additional Lab Tests

Hemoglobin A1C
Uric Acid

**Claims**

1. A composition comprising (a) a therapeutically effective amount of calcium alpha-ketoglutarate, and (b) a controlled-release matrix; wherein the controlled-release matrix comprises an hydroxypropylmethylcellulose (HPMC) with number average molecular weight of about 68,000-95,000; or an HPMC with number average molecular weight of about 115,000-150,000; or typically wherein the controlled-release matrix comprises a first HPMC with number average molecular weight between about 68,000-95,000 and a second HPMC with number average molecular weight between about 115,000-150,000.

2. The composition according to claim 1 wherein the amount of calcium alpha-ketoglutarate is from about 30% to about 65% by total weight of the composition.

3. The composition according to claim 1 or claim 2 comprising about 250 to about 1000 mg of calcium alpha-ketoglutarate; or typically about 350 to about 1000 mg of calcium alpha-ketoglutarate; or typically about 400 to about 600 mg of calcium alpha-ketoglutarate; or typically about 525 mg of calcium alpha-ketoglutarate.

4. The composition according to any one of claims 1-3 comprising at least about 10% dietary value of calcium; or typically at least about 15% dietary value of calcium.

5. The composition according to any one of claims 1-4 further comprising one or more excipients; typically further comprising microcrystalline cellulose; or typically further comprising lactose monohydrate; or typically further comprising microcrystalline cellulose and lactose monohydrate.

6. The composition according to any one of claims 1-5 further comprising one or more lubricants; typically wherein the lubricant is magnesium stearate.

7. The composition according to any one of claims 1-6 further comprising one or more disintegrants; typically wherein the disintegrant is silicon dioxide.

8. The composition according to any one of claims 1-7 further comprising a sweetener; typically wherein the sweetener is isomalt.

9. The composition according to any one of claims 1-8 further comprising wax; typically wherein the wax is carnauba wax, rice bran wax, or a combination of carnauba wax and rice bran wax.

10. The composition according to any one of claims 1-9 wherein the controlled-release matrix comprises a first HPMC with

number average molecular weight between about 68,000-95,000 and a second HPMC with number average molecular weight between about 115,000-150,000; and **characterized by** a release rate of the calcium alpha-ketoglutarate that is about 90% or less of a release rate of calcium alpha-ketoglutarate of a calcium alpha-ketoglutarate composition that is uncontrolled or is formulated without the controlled-release matrix; typically **characterized by** a release rate of the calcium alpha-ketoglutarate that is about 70% or less of a release rate of calcium alpha-ketoglutarate of a calcium alpha-ketoglutarate composition that is uncontrolled or is formulated without the controlled-release matrix; or more typically **characterized by** a release rate of the calcium alpha-ketoglutarate that is about 60% or less of a release rate of calcium alpha-ketoglutarate of a calcium alpha-ketoglutarate composition that is uncontrolled or is formulated without the controlled-release matrix; or more typically **characterized by** a release rate of the calcium alpha-ketoglutarate that is about 50% or less of a release rate of calcium alpha-ketoglutarate of a calcium alpha-ketoglutarate composition that is uncontrolled or is formulated without the controlled-release matrix.

**11.** The composition according to any one of claims 1-10 wherein the controlled-release matrix comprises a first HPMC with number average molecular weight between about 68,000-95,000 and a second HPMC with number average molecular weight between about 115,000-150,000; and **characterized by** a release rate of the calcium alpha-ketoglutarate that is about 30% or less after a time between about 0.5 and about 2 hours in a dissolution medium as measured by a paddle apparatus with a basket rotating at 75 rpm, wherein the dissolution medium is 900 mL of about 0.03 M NaCl and about 0.08 M HCl at about 37°C.

**12.** The composition according to any one of claims 1-11 wherein the controlled-release matrix comprises a first HPMC with number average molecular weight between about 68,000-95,000 and a second HPMC with number average molecular weight between about 115,000-150,000; and **characterized by** a release rate of the calcium alpha-ketoglutarate that is about 75% or more after a time between about 10 and about 11 hours in a dissolution medium as measured by a paddle apparatus with a basket rotating at 75 rpm, wherein the dissolution medium is 900 mL of about 0.03 M NaCl and about 0.08 M HCl at about 37°C.

**13.** The composition according to any one of claims 1-12 wherein the calcium alpha-ketoglutarate is calcium alpha-ketoglutarate monohydrate.

**14.** The composition according to any one of claims 1-13 in the form of a dosage unit; typically in the form of a dosage unit configured for oral administration; typically in the form of a tablet; or optionally in the form of a capsule.

**15.** A tablet comprising 525 mg of calcium alpha-ketoglutarate monohydrate, and an HPMC with number average molecular weight of about 68,000-95,000 or an HPMC with number average molecular weight of about 115,000-150,000; or typically a first HPMC with number average molecular weight between about 68,000-95,000 and a second HPMC with number average molecular weight between about 115,000-150,000.

## Patentansprüche

**1.** Zusammensetzung, umfassend (a) eine therapeutisch wirksame Menge an Calcium-Alpha-Ketoglutarat, und (b) eine Matrix zur gesteuerten Freisetzung; wobei die Matrix zur gesteuerten Freisetzung eine Hydroxypropylmethylcellulose (HPMC) mit zahlenmittlerem Molekulargewicht von etwa 68.000-95.000; oder eine HPMC mit zahlenmittlerem Molekulargewicht von etwa 115.000-150.000 umfasst; oder typischerweise wobei die Matrix zur gesteuerten Freisetzung eine erste HPMC mit zahlenmittlerem Molekulargewicht zwischen etwa 68.000-95.000 und eine zweite HPMC mit zahlenmittlerem Molekulargewicht zwischen etwa 115.000-150.000 umfasst.

**2.** Zusammensetzung nach Anspruch 1, wobei die Menge an Calcium-Alpha-Ketoglutarat von etwa 30% bis etwa 65% des Gesamtgewichts der Zusammensetzung beträgt.

**3.** Zusammensetzung nach Anspruch 1 oder Anspruch 2, umfassend etwa 250 bis etwa 1000 mg Calcium-Alpha-Ketoglutarat; oder typischerweise etwa 350 bis etwa 1000 mg Calcium-Alpha-Ketoglutarat; oder typischerweise etwa 400 bis etwa 600 mg Calcium-Alpha-Ketoglutarat; oder typischerweise etwa 525 mg Calcium-Alpha-Ketoglutarat.

**4.** Zusammensetzung nach einem der Ansprüche 1-3, umfassend mindestens etwa 10% Nährwert an Calcium; oder typischerweise mindestens etwa 15% Nährwert an Calcium.

**5.** Zusammensetzung nach einem der Ansprüche 1-4, weiter umfassend einen oder mehrere Hilfsstoffe; typischerweise weiter umfassend mikrokristalline Cellulose; oder typischerweise weiter umfassend Lactose-Monohydrat; oder typischerweise weiter umfassend mikrokristalline Cellulose und Lactose-Monohydrat.

**6.** Zusammensetzung nach einem der Ansprüche 1-5, weiter umfassend ein oder mehrere Schmiermittel; typischerweise wobei das Schmiermittel Magnesiumstearat ist.

**7.** Zusammensetzung nach einem der Ansprüche 1-6, weiter umfassend ein oder mehrere Sprengmittel; typischerweise wobei das Sprengmittel Siliziumdioxid ist.

**8.** Zusammensetzung nach einem der Ansprüche 1-7, weiter umfassend einen oder mehrere Süßstoffe; typischerweise wobei der Süßstoff Isomalt ist.

**9.** Zusammensetzung nach einem der Ansprüche 1-8, weiter umfassend Wachs; typischerweise wobei das Wachs Carnaubawachs, Reiskleiewachs oder eine Kombination aus Carnaubawachs und Reiskleiewachs ist.

**10.** Zusammensetzung nach einem der Ansprüche 1-9, wobei die Matrix zur gesteuerten Freisetzung eine erste HPMC mit zahlenmittlerem Molekulargewicht zwischen etwa 68.000-95.000 und eine zweite HPMC mit zahlenmittlerem Molekulargewicht zwischen etwa 115.000-150.000 umfasst; und **gekennzeichnet durch** eine Freisetzungsrate des Calcium-Alpha-Ketoglutarats, die etwa 90% oder weniger als eine Freisetzungsrate von Calcium-Alpha-Ketoglutarat einer Calcium-Alpha-Ketoglutarat-Zusammensetzung beträgt, die nicht gesteuert ist oder ohne die Matrix zur gesteuerten Freisetzung formuliert ist; typischerweise **gekennzeichnet durch** eine Freisetzungsrate des Calcium-Alpha-Ketoglutarats, die etwa 70% oder weniger als eine Freisetzungsrate von Calcium-Alpha-Ketoglutarat einer Calcium-Alpha-Ketoglutarat-Zusammensetzung beträgt, die nicht gesteuert ist oder ohne die Matrix zur gesteuerten Freisetzung formuliert ist; oder noch typischerweise **gekennzeichnet durch** eine Freisetzungsrate des Calcium-Alpha-Ketoglutarats, die etwa 60% oder weniger als eine Freisetzungsrate von Calcium-Alpha-Ketoglutarat einer Calcium-Alpha-Ketoglutarat-Zusammensetzung beträgt, die nicht gesteuert ist oder ohne die Matrix zur gesteuerten Freisetzung formuliert ist; oder noch typischerweise **gekennzeichnet durch** eine Freisetzungsrate des Calcium-Alpha-Ketoglutarats, die etwa 50% oder weniger als eine Freisetzungsrate von Calcium-Alpha-Ketoglutarat einer Calcium-Alpha-Ketoglutarat-Zusammensetzung beträgt, die nicht gesteuert ist oder ohne die Matrix zur gesteuerten Freisetzung formuliert ist.

**11.** Zusammensetzung nach einem der Ansprüche 1-10, wobei die Matrix zur gesteuerten Freisetzung eine erste HPMC mit zahlenmittlerem Molekulargewicht zwischen etwa 68.000-95.000 und eine zweite HPMC mit zahlenmittlerem Molekulargewicht zwischen etwa 115.000-150.000 umfasst; und **gekennzeichnet durch** eine Freisetzungsrate des Calcium-Alpha-Ketoglutarats, die nach einer Zeit zwischen etwa 0,5 und etwa 2 Stunden in einem Auflösungsmedium, wie durch eine Paddeleinrichtung mit einem Korb, der bei 75 U/min rotiert, gemessen, etwa 30% oder weniger beträgt, wobei das Auflösungsmedium 900 ml von etwa 0,03 M NaCl und etwa 0,08 M HCl bei etwa 37°C ist.

**12.** Zusammensetzung nach einem der Ansprüche 1-11, wobei die Matrix zur gesteuerten Freisetzung eine erste HPMC mit zahlenmittlerem Molekulargewicht zwischen etwa 68.000-95.000 und eine zweite HPMC mit zahlenmittlerem Molekulargewicht zwischen etwa 115.000-150.000 umfasst; und **gekennzeichnet durch** eine Freisetzungsrate des Calcium-Alpha-Ketoglutarats, die nach einer Zeit zwischen etwa 10 und etwa 11 Stunden in einem Auflösungsmedium, wie durch eine Paddeleinrichtung mit einem Korb, der bei 75 U/min rotiert, gemessen, etwa 75% oder weniger beträgt, wobei das Auflösungsmedium 900 ml von etwa 0,03 M NaCl und etwa 0,08 M HCl bei etwa 37°C ist.

**13.** Zusammensetzung nach einem der Ansprüche 1-12, wobei das Calcium-Alpha-Ketoglutarat Calcium-Alpha-Ketoglutarat-Monohydrat ist.

**14.** Zusammensetzung nach einem der Ansprüche 1-13 in der Form einer Dosierungseinheit; typischerweise in der Form einer Dosierungseinheit, die zur oralen Verabreichung konfiguriert ist; typischerweise in der Form einer Tablette; oder optional in der Form einer Kapsel.

**15.** Tablette, umfassend 525 mg Calcium-Alpha-Ketoglutarat-Monohydrat, und eine HPMC mit zahlenmittlerem Molekulargewicht von etwa 68.000-95.000 oder eine HPMC mit zahlenmittlerem Molekulargewicht von etwa 115.000-150.000; oder typischerweise eine erste HPMC mit zahlenmittlerem Molekulargewicht zwischen etwa 68.000-95.000 und eine zweite HPMC mit zahlenmittlerem Molekulargewicht zwischen etwa 115.000-150.000.

## Revendications

1. Composition comprenant (a) une quantité thérapeutiquement efficace d'alpha-cétoglutarate de calcium, et (b) une matrice à libération régulée ; dans laquelle la matrice à libération régulée comprend une hydroxypropylméthylcellulose (HPMC) avec un poids moléculaire moyen en nombre d'environ 68 000-95 000 ; ou une HPMC avec un poids moléculaire moyen en nombre d'environ 115 000-150 000 ; ou généralement dans laquelle la matrice à libération régulée comprend une première HPMC avec un poids moléculaire moyen en nombre entre environ 68 000-95 000 et une seconde HPMC avec un poids moléculaire moyen en nombre entre environ 115 000-150 000.

2. Composition selon la revendication 1, dans laquelle la quantité d'alpha-cétoglutarate de calcium est d'environ 30 % à environ 65 % en poids total de la composition.

3. Composition selon la revendication 1 ou la revendication 2 comprenant environ 250 à environ 1000 mg d'alpha-cétoglutarate de calcium ; ou généralement environ 350 à environ 1000 mg d'alpha-cétoglutarate de calcium ; ou généralement environ 400 à environ 600 mg d'alpha-cétoglutarate de calcium ; ou généralement environ 525 mg d'alpha-cétoglutarate de calcium.

4. Composition selon l'une quelconque des revendications 1 à 3 comprenant au moins environ 10 % de valeur nutritionnelle de calcium ; ou généralement au moins environ 15 % de valeur nutritionnelle de calcium.

5. Composition selon l'une quelconque des revendications 1 à 4 comprenant en outre un ou plusieurs excipients ; comprenant en outre, généralement, de la cellulose microcristalline ; ou comprenant en outre, généralement, du lactose monohydraté ; ou comprenant en outre, généralement, de la cellulose microcristalline et du lactose monohydraté.

6. Composition selon l'une quelconque des revendications 1 à 5 comprenant en outre un ou plusieurs lubrifiants ; généralement dans laquelle le lubrifiant est du stéarate de magnésium.

7. Composition selon l'une quelconque des revendications 1 à 6, comprenant en outre un ou plusieurs délitants ; généralement dans laquelle le délitant est le dioxyde de silicium.

8. Composition selon l'une quelconque des revendications 1 à 7 comprenant en outre un édulcorant ; généralement dans laquelle l'édulcorant est l'isomalt.

9. Composition selon l'une quelconque des revendications 1 à 8, comprenant en outre une cire ; généralement dans laquelle la cire est de la cire de carnauba, de la cire de son de riz ou une combinaison de cire de carnauba et de cire de son de riz.

10. Composition selon l'une quelconque des revendications 1 à 9, dans laquelle la matrice à libération régulée comprend une première HPMC avec un poids moléculaire moyen en nombre entre environ 68 000-95 000 et une seconde HPMC avec un poids moléculaire moyen en nombre entre environ 115 000-150 000 ; et **caractérisée par** une vitesse de libération de l'alpha-cétoglutarate de calcium qui est d'environ 90 % ou moins d'une vitesse de libération d'alpha-cétoglutarate de calcium d'une composition d'alpha-cétoglutarate de calcium qui n'est pas régulée ou qui est formulée sans la matrice à libération régulée ; généralement, **caractérisée par** une vitesse de libération de l'alpha-cétoglutarate de calcium qui est d'environ 70 % ou moins d'une vitesse de libération d'alpha-cétoglutarate de calcium d'une composition d'alpha-cétoglutarate de calcium qui n'est pas régulée ou qui est formulée sans la matrice à libération régulée ; ou plus généralement, **caractérisée par** une vitesse de libération de l'alpha-cétoglutarate de calcium qui est d'environ 60 % ou moins d'une vitesse de libération d'alpha-cétoglutarate de calcium d'une composition d'alpha-cétoglutarate de calcium qui n'est pas régulée ou qui est formulée sans la matrice à libération régulée ; ou plus généralement, **caractérisée par** une vitesse de libération de l'alpha-cétoglutarate de calcium qui est d'environ 50 % ou moins d'une vitesse de libération d'alpha-cétoglutarate de calcium d'une composition d'alpha-cétoglutarate de calcium qui est non régulée ou qui est formulée sans la matrice à libération régulée.

11. Composition selon l'une quelconque des revendications 1 à 10, dans laquelle la matrice à libération régulée comprend une première HPMC avec un poids moléculaire moyen en nombre entre environ 68 000-95 000 et une seconde HPMC avec un poids moléculaire moyen en nombre entre environ 115 000-150 000 ; et **caractérisée par** une vitesse de libération de l'alpha-cétoglutarate de calcium qui est d'environ 30 % ou moins après un temps entre environ 0,5 et environ 2 heures dans un milieu de dissolution, telle que mesurée par un appareil à palettes avec un

panier tournant à 75 tr/min, dans laquelle le milieu de dissolution est 900 ml d'environ 0,03 M de NaCl et d'environ 0,08 M de HCl à environ 37 °C.

**12.** Composition selon l'une quelconque des revendications 1 à 11, dans laquelle la matrice à libération régulée comprend une première HPMC avec un poids moléculaire moyen en nombre entre environ 68 000-95 000 et une seconde HPMC avec un poids moléculaire moyen en nombre entre environ 115 000-150 000 ; et **caractérisée par** une vitesse de libération de l'alpha-cétoglutarate de calcium qui est d'environ 75 % ou plus après un temps entre environ 10 et environ 11 heures dans un milieu de dissolution, telle que mesurée par un appareil à palettes avec un panier tournant à 75 tr/min, dans laquelle le milieu de dissolution est 900 ml d'environ 0,03 M de NaCl et d'environ 0,08 M de HCl à environ 37 °C.

**13.** Composition selon l'une quelconque des revendications 1 à 12, dans laquelle l'alpha-cétoglutarate de calcium est l'alpha-cétoglutarate de calcium monohydraté.

**14.** Composition selon l'une quelconque des revendications 1 à 13 sous forme posologique unitaire ; généralement sous forme posologique unitaire configurée pour une administration orale ; généralement sous forme de comprimé ; ou facultativement sous forme de capsule.

**15.** Comprimé comprenant 525 mg d'alpha-cétoglutarate de calcium monohydraté, et une HPMC avec un poids moléculaire moyen en nombre d'environ 68 000-95 000 ou une HPMC avec un poids moléculaire moyen en nombre d'environ 115 000-150 000 ; ou généralement une première HPMC avec un poids moléculaire moyen en nombre d'environ 68 000-95 000 et une seconde HPMC avec un poids moléculaire moyen en nombre d'environ 115 000-150 000.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018200736 A
- US 2776926 A


### Non-patent literature cited in the description


- **MACKENZIE et al.** *Mol Cell Biol*, 2007, vol. 27 (9), 3282-3289
- **BUNIK et al.** *Biochemistry*, 2005, vol. 44 (31), 10552-61
- Handbook of Pharmaceutical Salts: Properties, Selection and Use. International Union of Pure and Applied Chemistry. Wiley-VCR, 2002
- **S.M. BERGE** ; **L.D. BIGHLEY** ; **D.C. MONKHOUSE**. *J. Pharm. Sci.*, 1977, vol. 66, 1-19
- Handbook of Pharmaceutical Salts: Properties, Selection and Use. Wiley-VCH/VHCA, 2002
- Remington's Pharmaceutical Sciences. Mack Publishing Co.
- Remington: The Science and Practice of Pharmacy. Mack Publishing Company, 1995
- **HOOVER, JOHN E.** Remington's Pharmaceutical Sciences. Mack Publishing Co., 1975
- Pharmaceutical Dosage Forms, Marcel Decker. 1980
- **LIPPINCOTT WILLIAMS** ; **WILKINS**. Pharmaceutical Dosage Forms and Drug Delivery Systems. 1999
- **HOOVER** ; **JOHN E.** Remington's Pharmaceutical Sciences. Mack Publishing Co., 1975
- Pharmaceutical Dosage Forms. Marcel Decker, 1980
- **LUCANIC, M.** ; **LITHGOW, G. J.** ; **& ALAVEZ, S.** Pharmacological lifespan extension of invertebrates. *Ageing research reviews*, 2013, vol. 12 (1), 445-458
- **SEARLE, S. D.** ; **MITNITSKI, A.** ; **GAHBAUER, E. A.** ; **GILL, T. M.** ; **ROCKWOOD, K.** A standard procedure for creating a frailty index. *BMC geriatrics*, 2008, vol. 8 (1), 24
- **BELSKY, D. W.** ; **CASPI, A.** ; **HOUTS, R.** ; **COHEN, H. J.** ; **CORCORAN, D. L.** ; **DANESE, A.** ; **SUGDEN, K.** Quantification of biological aging in young adults. *Proceedings of the National Academy of Sciences*, 2015, vol. 112 (30), E4104-E4110